# EUROPEAN PATENT SPECIFICATION

(11) **EP 1 214 911 B1**
(45) Date of publication and mention of the grant of the patent: **28.12.2005**
(21) Application number: 01129309.9
(22) Date of filing: 12.05.2000
(51) Int. Cl.: A61B 17/00

(54) **Septal defect closure device**
Verschlusseinrichtung eines Septumschadens
Dispositif de fermeture de malformation septale

(30) Priority: 13.05.1999 US 134250 P
(43) Date of publication of application: 19.06.2002
(62) Divisional of application: 00930666.3
(73) Proprietor: St. Jude Medical ATG, Inc., Maple Grove, MN 55311 (US)
(72) Inventor: Berg, Todd Allen, Plymouth MN 55442 (US); Peterson, Alex Alden, Maple Grove MN 55311 (US); Swanson, William J., St Paul MN 55108 (US); Hindrichs, Paul J., Plymouth MN 55441 (US)
(74) Representative: Vossius & Partner

(56) References cited:
- EP-A- 0 541 063
- WO-A-93/13712
- WO-A-97/41779
- WO-A-98/01086
- WO-A-98/08462
- WO-A-98/27868
- WO-A-99/07289
- WO-A-99/38454
- DE-A- 2 822 603
- US-A- 5 853 422

## Description

### Background of the Invention

This invention relates to apparatus for closing intravascular defects and occluding blood flow. In particular, it relates to closing septal defects or holes found between the walls of the four heart chambers and occluding blood flow in sections of a patient's circulatory system.

The heart chambers include left and right atrial chambers in the upper portion and left and right ventricular chambers in the lower portion. Defects in these walls can be formed congenitally or can develop later in life. An atrial septal defect (hereinafter, "ASD") is found between the right and left atrium and a ventricular septal defect (hereinafter, "VSD") is found between the left and right ventricles. The defect allows blood to be shunted between the chambers, causing the heart's pumping action to be inefficient, and creating a risk of embolization (the circulation of an abnormal parcicle through the bloodstream)

A similar defect is the patent ductus. The patent ductus is a pre-birth opening between the aorta and the pulmonary arzery. This opening usually closes naturally, but may remain open and cause oxygenated blood to flow back into the lungs. Other defects are the ductus arteriosus and the patent foramen ovale (hereinafter, "PFO"). At least fifty percent of stroke patients under 55 years old have a PFO.

Therapeutic treatment of these defects normally requires expensive surgery. For example, treatment typically requires open heart surgery, cardiopulmonary bypass, and shopping of the heart. During treatment, the defect is sewn shut by applying a thin patch over the hole. Less invasive methods for closure of these defects such as intraluminal transcatheter approaches, for example, may be used, but provide unreliable delivery and deployment. A transcatheter apparatus has a large delivery profile that limits application of the method to young patients and makes it difficult to match the apparatus to the intracardiac or extracardiac cavity and can result in thrombosis, emboli, or dislodgement due to interference with blood flow.

In WO 99/38454, which form prior art pursuant Article 54(3) EPC, a plug is diclosed provided with fingers which extend axially from each end of a medial portion of the plug. The fingers are deflected radially outwardly from the medial portion and set in that condition. For a graft connector, the medial portion is coaxially connected to an end portion of a tubular graft. The connector is then installed through an aperture in the side wall of a patient's tubular conduit, e.g. by using a delivery tube in which the fingers are elastically deflected back to approximately their initial positions. When the delivery conduit is withdrawn, the fingers spring out to engage the inner and outer surfaces of the body conduit wall.

It is also known that septal holes cause strokes by shunting clots from the right atrium to the left atrium. From the left atrium, a clot can go to the brain. Some holes are asymptomatic and should still be closed to prevent future stroke. Patients having asymptomatic defects would benefit from a low-invasive and reliable treatment apparatus and method.

In addition to the treatment of septal defects, it is often desirable occlude blood flow in a section of the circulatory system. Occlusion can control internal bleeding and buffer pressure in the vicinity of an aneurysm.

Therefore, it would be desirable to provide apparatus and methods for treating septal defects, such as ASD, VSD; and PFO, that function at least as well as the proven surgical thin sewn patch, but which are less invasive.

It would also be desirable to provide reliable apparatus and methods for delivery of intraluminal transcatheters and deployment of septal defect devices and plugs.

It would be more desirable to provide these apparatus and methods such that the delivery profile is small and such that they can be used to treat patients of a wide range of ages.

It would be further desirable to provide septal defect devices and occluding plugs that can be properly matched to the intracardiac or extracardiac cavity.

It would be still further desirable to provide apparatus and methods for percutaneous delivery and deployment of occlusion devices for blocking blood flow in various sections of the circulatory system.

### Summary of the Invention

Therefore, it is an object of the invention to provide apparatus for treating septal defects, such as ASD, VSD, and PFO, that function as well as the proven surgical thin sewn patch, but which are less invasive.

Additionally, it is an object of the invention to provide these apparatus such that the delivery profile is small and such that they can be used to treat patients of having a wide range of ages.

It is a further object of the invention to provide septal defect devices and occluding plugs that can be properly matched to the intracardiac or extracardiac cavity.

According to the invention as defined in claim 1, a plug is provided that has a perforated tubular portion having a longitudinal passage. Any cross-sertion of the perforated tubular portion taken along a plane perpendicular to the passage is discontinuous to allow conformation of the portion to perimeter of an aperture. The plug has a plurality of fingers expending from each of the two axial ends of the perforated tubular portion. Any cross-section of the fingers taken along a plane perpendicular to the passage is also discontinuous. The plug also has plugging structure.

In another aspect of the invention, an occlusive device is provided for occluding blood flow at a treatment site. This device is similar to the preceding plug, but has fingers expending from only one axial end of the perforated tubular portion. During operation, these fingers anchor the device to the internal surface of a blood vessel.

### Brief Description of the Drawings

The above and other objects and advantages of the invention will be apparent upon consideration of the following detailed description, taken in conjunction with the accompanying drawings, in which like reference characters refer to like parts throughout, and in which:
FIG. 1 is a plan view of a plug for plugging a septal defect not in accordance with this invention;
FIG. 1A is a partial side elevational view along direction 1A-1A of FIG. 1 not in accordance with the principles of this invention;
FIG. 2 is a side elevational view of a finger of a plug not in accordance with this invention;
FIG. 3 is a side elevational view of another finger of a plug not in accordance with this invention;
FIG. 4 is a side elevational view of yet another finger of a plug not in accordance with this invention;
FIG. 5 is a side elevational view of still another finger of a plug not in accordance with this invention;
FIG. 6 is a plan view of another plug for plugging a septal defect not in accordance with this invention;
FIG. 7 is a side elevational view of yet another finger of a plug not in accordance with this invention;
FIG. 8 is a plan view of yet another plug for plugging a septal defect not in accordance with this invention;
FIG. 9 is a plan view of yet another plug for plugging a septal defect not in accordance with this invention;
FIG. 10 is a cross-sectional view of a plug for plugging a septal defect disposed within a delivery device not in accordance with this invention;
FIG. 11 is a cross-sectional view of the plug shown in FIG. 10 when the plug is partially deployed in the septal defect not in accordance with this invention;
FIG. 12 is a cross-sectional view of the plug shown in FIG. 10 and 11 when the plug is fully deployed in the septal defect not in accordance with this invention;
FIG 13 is a cross-sectional view of a plug similar to the one shown in FIGS. 10-12 when the plug is fully deployed showing the delivery path of a delivery device not in accordance with this invention;
FIG. 14 is a cross-sectional view of the plug shown in FIG. 13 for plugging a septal defect when the plug is fully deployed and the delivery device has been retracted from the heart not in accordance with this invention;
FIG. 15 is an elevational view of the plug shown in FIGS. 13 and 14 taken along line 15-15 of FIG. 14 not in accordance with this invention:
FIG. 16 is a plan view of yet another plug for plugging a septal defect not in accordance with this invention;
FIG. 17 is a plan view of the plug shown in FIG. 16 from the opposite side not in accordance with this invention
FIG. 18 is a cross-sectional view of the plug shown in FIGS. 16 and 17 when the plug is fully deployed in the septal defect not in accordance with this invention;
FIG. 19 is a plan view of the plug shown in FIGS. 16-18 taken from line 19-19 of FIG. 18 not in accordance with this invention;
FIG. 20 is a partial elevational view of an unrolled frame for an illustrative plug in accordance with this invention;
FIG. 21 is a perspective view of the of the frame shown in FIG. 21 with ends attached (showing only a single finger at each axial end of the frame) in accordance with this invention;
FIG. 22 is a cross-sectional view of the frame shown in FIGS. 20 and 21 with ends attached in accordance with this invention:
FIG. 23 is an elevational view of the frame shown in FIG. 22 in accordance with this invention
FIG. 24 is a perspective view of the frame shown in FIG. 21 with an attached plugging structure to form a plug in accordance with this invention;
FIG. 25 is an elevational view of the plug shown in FIG. 24 (showing a number of forward facing fingers) after the fingers have been bent into the wall-engaging position in accordance with this invention;
FIG. 26 is a partial elevational view of an unrolled frame with a plugging structure in accordance with this invention;
FIG. 27 is an elevational view of another unrolled frame for a plug in accordance with this invention;
FIG. 28 is an elevational view of another plug mounted on a delivery balloon in accordance with this invention;
FIG. 29 is a cross-sectional view taken of the plug shown in FIG. 28 in position for deployment in a defect in accordance with this invention;
FIG. 30 is a cross-sectional view of the plug shown in FIGS. 28 and 29 when the plug is deployed in the defect in accordance with this invention;
FIG. 31 is a cross-sectional view of the plug shown in FIGS. 28-30 in position for deployment showing the delivery path of a delivery device in accordance with this invention;
FIG. 32 is a cross-sectional view of yet another plug for plugging a septal defect when the plug is fully deployed in the septal defect in accordance with this invention;
FIG. 33 is a partial elevational view of an unrolled frame for an occlusion device (i.e., a plug) in accordance with this invention;
FIG. 34 is an elevational view of another occlusion device with barbs mounted on a delivery balloon in accordance with this invention;
FIG. 35 is a partial elevational view of another occlusion device for occluding a section of a blood vessel in accordance with this invention; and
FIG. 36 is a cross-sectional view of the occlusion device shown in FIG. 35 deployed in a blood vessel in accordance with this invention.

### Detailed Description of the Preferred Embodiments

Apparatus and methods for preventing the flow of body fluids through apertures in body cavity walls and through a patient's body tubing, such as a blood vessel, can be a plug that is installed in the patient's body using an intraluminal catheter method. The plug can have a (1) frame that conforms to the walls of an aperture (e.g., a defect) or a section of tubing and (2) a plugging structure (e.g., a patch) that prevents the flow of fluid. Although the plug can be installed in a variety of types of body tissues to prevent the flow of body fluid, only related to preventing the flow of blood through passageways in the circulatory system will be illustrated wherein.

In one form, a plug is provided for closing an aperture or hole in a septal wall of a patient's heart, for example a PFO. The plug has a frame, two pluralities of fingers attached to axial ends of the frame and to each other, and a plugging structure attached thereto. The pluralities of fingers can be integral with the frame and formed from a unitary body. During operation, one plurality of fingers engages an interior surface of the wall and the other set of fingers engages the opposite, or exterior, surface. The plugging structure is supported by the frame and spans the aperture (e.g., defect) to prevent the flow of blood therethrough.

The fingers are preferably positioned substantially circumferentially (i.e., peripherally) about the plug' s central axis, which passes through the flame The fingers can extend radially away or along the axis. The fingers have ends that are radially proximal to the central axis and which generally define a substantially round or elliptical broken cross-section in a plane substantially perpendicular to the central axis.

Preferably, any cross section of the frame that lies in a plane substantially perpendicular to the axis is substantially discontinuous. This allows the frame to contract and expand radially as necessary for insertion into a delivery device, placement in an aperture, and conformation to the walls of the aperture. The frame itself can comprise an elastic material, such as nitinol. Alternatively, the frame can comprise a plastically deforming material, such as stainless steel. The elastic and plastic embodiments may be delivered differently.

When the plug is inside the patient's body medical scanners can be used to assist and confirm plug placement and to evaluate the integrity of a plug after it has been in use for an extended period of time. The frame may be equipped with one or more marker structures which can be radiopaque, to help identify, locate, and orient the plug using images produced with, for example, X-rays, CT scans, ultrasound, and echo techniques.

Marker structures can be provided in a variety of forms. For example, a marker structure can be in the form of a marker band made from a radiopaque material that is crimped onto an end portion of a finger. Alternatively, a marker structure can be a rivet that is inserted and locked into a ring or hole in a finger. If marker structures are used, they can be provided on any number of fingers.

Other structures can be present on the fingers of the plug to facilitate its delivery. For example, a finger can be provided with a retention device receptacle. A retention device reciprocates within a delivery sleeve or catheter and engages the fingers while the plug is inserted in the delivery sleeve so that the plug can be reciprocated within the sleeve and shifted into position in the aperture, such as a PFO. Once in position, the retention device can release the fingers so that the fingers spring into engagement with the aperture wall.

In one form, the frame of the plug is insertable into a delivery tube by extending the fingers in a direction substantially parallel to the central axis. The retention device itself has fingers or locking pins that engage the retention device receptacles that reside on the ends of the frame fingers. Retention device receptacles include, but are not limited to locking pin apertures and nose cone covers.

Fingers may have a variety of designs that are tailored to optimize plug security for the shape and tissue characteristics of a given PFO. For example, fingers can have pointed ends, barbs, or curved portions. In one embodiment, fingers can be curved toward a plane that is perpendicular to the central axis and that passes substantially between the two pluralities of fingers. Fingers can be of substantially similar length or substantially different lengths. Any finger can have different flexural stiffness at different points along its length. One way to accomplish differential flexural stiffness of a finger is to provide a finger having a different thickness or a different width at different points along its length. Alternatively, both the finger thickness and the finger width can vary along the length of a given finger, if desired.

In certain cases, the force applied by a finger to the septum wall can be distributed to minimize stress concentration in the wall. In that case, the plug can be provided with an elastic web supported between adjacent fingers. The web, for example, can include silicone.

The plugging structure that occludes the PFO can be attached to or supported by the frame at proximal or distal ends of the fingers. In either case, the fingers' ends can be provided with support structures with which the plugging structure can be affixed.

In one embodiment, the plugging structure is made from an elastic material and can contract and expand as the frame contracts and expands (e.g., during delivery and deployment). One material that can be used to make the plugging structure is polyester (such as the material sold under the trademark DACRON® by E.I. du Pont de Nemours & Company of Wilmington, Delaware.).

The plugging structure can also be made from cloth and be folded and unfolded as the plug is contracted and expanded as may be necessary for its installation in the defect. In either the elastic or cloth embodiments of the plugging structure, the plugging structure can be attached (e.g., sewn) directly to the frame. The plugging structure can have a guide wire aperture through which a guide wire can pass. If the guide wire is inserted into the patient prior to plug delivery, the guide wire can be used to guide the plug into place in the PFO. The guide wire aperture can be designed to substantially self close after the guide wire is removed from the guide wire aperture. The self-closing feature can be achieved by making the diameter of a guide wire aperture in the relaxed state (i.e., without the wire) small enough to induce clotting and close off blood flow.

In an embodiment of the invention, the plug has a perforated tubular portion that forms a longitudinal passage. Fingers extend from each of the two axial ends of the perforated tubular portion and may be provided in a variety of configurations and made from a variety of materials. Any of the features discussed above may also be included. Like the frames discussed above, the perforated tubular portion is discontinuous along any cross section taken in a plane perpendicular to its longitudinal axis. This feature permits the perforated tubular portion to contract and expand radially and longitudinally for delivery, deployment, and conformation to the walls adjacent a PFO, for example. Similarly, the plurality of fingers are discontinuous along any cross section taken in a plane perpendicular to the longitudinal axis to allow such contraction and expansion as well.

The plugging structure can be supported directly or indirectly by the perforated tubular portion. For example, the plugging structure can be attached directly to the perforated tubular portion or to elements of the structure (such as tabs, bosses, extensions, or loops). Alternatively, the plugging structure can be attached via interceding support structures (such as attachment rings, clips, or loops) that connect the tubular portion to the plugging structure.

The perforated tubular portion preferably contracts longitudinally as it expands radially. The tubular portion can be made of a material that deforms plastically or elastically. A plastically deforming material can be, for example, stainless steel or tantalum. The plug is installed by positioning the plug in the aperture of the PFO and expanding a balloon inside the plug to at least partially conform the perforated tubular portion to the perimeter of the aperture. The perforations allow the plug to contract longitudinally in response to the radial expansion. The longitudinal contraction causes the fingers to engage opposing sides of the wall.

According to another aspect of the invention an occlusion plug is provided. The occlusion plug has a perforated tubular portion for occluding a blood vessel. This type of plug may be desirable to prevent blood flow near a damaged portion of the vessel (e.g., aneurysm) The occlusion plug can be plastically or elastically deformable.

In the plastic embodiment the occlusion plug is installed in the blood vessel by expanding a balloon in a longitudinal passageway of the perforated tubular portion. The expansion of the balloon causes the plug to expand radially and contract longitudinally. This expansion causes the outer surface of the perforated tubular portion to conform to the inner surface of the lumen of the blood vessel. The expansion also causes the fingers at the end of the perforated tubular portion to engage the inner surface of the wall as they are driven radially outward from the longitudinal axis and drawn longitudinally toward the perforated tubular portion.

The fingers of the occlusion plug preferably extend from only one axial end of the perforated tubular portion. The configuration of the fingers, the structures associated with the fingers, the perforated tubular portion, and the relation of the fingers to the perforated tubular portion are similar to those described above in connection with the PFO plug.

The invention can be used in methods for preventing the flow of body fluids through apertures in body cavity walls. For simplicity plugging PFO's alone will be discussed. In a preferred method, a plug that is at least partially made from an elastic material and is conformable to a defect, such as any of those elastic plugs described above having two opposing pluralities of fingers is positioned at the defect, conformed to the perimeter of the defect and secured thereto.

In order to position the plug in the PFO, a delivery structure with a sleeve is provided. During the process of positioning the plug, the plug fingers are extended in a direction that is substantially parallel to the central axis of the plug while the plug is inserted into the sleeve of the delivery structure. A retention device inside the sleeve engages at least some of the extended fingers at the finger ends. The retention device can use locking pins, hooks, or any other means to retain the plug inside the sleeve. The retention elements permit an operator to reciprocate the plug longitudinally with respect to the sleeve and to shit the plug out from the end of the sleeve. The sleeve can be inserted like a catheter through an insertion aperture in a patient's body tissue. The sleeve can then be passed through the patient's internal body tubing or other body structures until the end of the sleeve is positioned within or adjacent the PFO for plug delivery

Once the end of the sleeve is near or within the PFO, the delivery structure can be shifted relative to the plug and the PFO, thereby removing the delivery structure from the PFO. The plug, however, extends through the PFO and the plug fingers extend outward, preferably radially, from the central axis of the plug to opposite sides of the wall in which the PFO resides. This causes the plug fingers to engage the wall and the plugging structure to substantially occlude the PF0.

Preferably, releasing the plug within the PF0 allows the plug to expand elastically inside the PF0 until the plug substantially conforms to the inner rim or perimeter of the PF0. In one method, the plug is allowed to elastically contract along the central axis of the plug while it expands radially. This longitudinal contraction causes the fingers to engage opposite sides of the same wall of the body cavity. A benefit of this approach is that the plug will center itself with respect to the wall in a direction along the plug's central axis (or along the longitudinal passage of the plug).

According to another method, a plastically deformable plug can be inserted in a PFO using a balloon. A conformable plug, such as any of those plastically deformable plugs described above, is positioned in the PFO, conformed to the perimeter of the PFO, and then secured thereto. The positioning can be achieved by inserting a delivery balloon into the tubular portion of the plug and delivering both through a patient's body tissue, (e.g., through an insertion aperture and blood vessels), to the PFO. Then, the balloon, which supports the plug, is moved through the patient's body until the plug is appropriately positioned in the aperture -- such that one plurality of fingers is situated on each side of the wall.

The plug conforms to the PFO when the balloon is expanded. This causes the fingers to engage on both sides of the septal wall. The tubular portion of the plug radially enlarges and conforms to the perimeter of the aperture and the plugging structure occludes the aperture. When a plug (such as any of those described above) is used according to this method, the balloon expansion plastically deforms the tubular portion. This expansion automatically causes the tubular portion to contract in the direction parallel to the central axis of the plug. (It will be appreciated that the tubular portion could be annular or have a ring-like arrangement of tabs or other elements). The axial contraction causes the plug to substantially center itself with respect to the wall and drives the fingers into both sides of the wall.

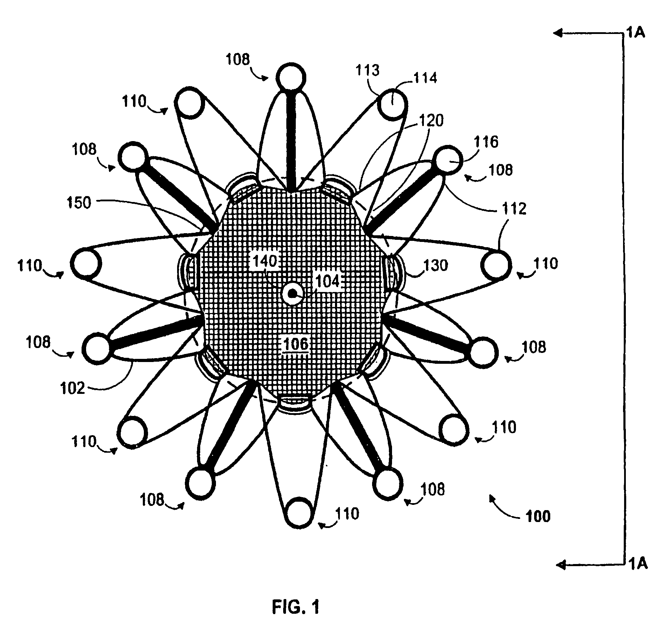

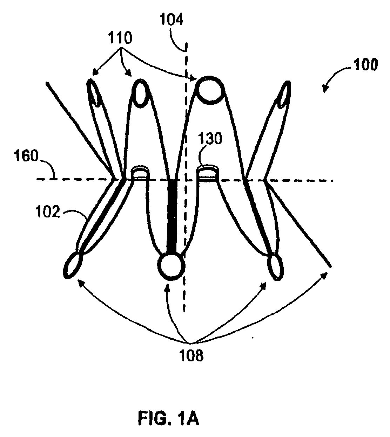

A number of apparatus, with several variations, are shown in FIGS. 1-36.

FIG. 1 shows plug 100 for closing an aperture, such as an ASD, a VSD, or a PFO, in a wall of a patient's body cavity. Frame 102 can be made from an elastic material, such as nickel titantium (hereinafter, "nitinol," available, for example, from Shape Memory Applications, of Santa Clara, California). The elastic nature of frame 102 allows frame 102 to radially contract sufficiently to allow it to be inserted into an aperture and subsequently radially expand to conform to the inner perimeter of the aperture. Other elastic materials can also be used to construct the frame and could be used in combination with other non-elastic materials. Radial expandability is facilitated by constructing the frame such that any cross section perpendicular to its central axis is discontinuous.

Frame 102 has central axis 104 and supports plugging structure 106. Frame 102 includes first plurality of fingers 108 and second plurality of fingers 110. In one form, fingers 108 are integral with fingers 110. Fingers 108 and 110 have proximal ends 120 that are near central axis 104 and remote ends 112 that are near the radially outer portions of frame 102.

Proximal ends 120 can be used to support plugging structure 106 directly, or they can be equipped with support structures 130 for supporting plugging structure 106. Alternatively, remote ends 112 can support plugging structure 106. Remote ends 112 can also be equipped with support structures 113 for supporting plugging structure 106, and, as discussed more fully below, marker devices. The ends can also be adapted to engage a retention device during plug installation. In FIG. 1, each support structure 113 has aperture 114 to which a plugging structure can be sewn or otherwise attached.

Plugging structure 106 can be made from an elastic material. Plugging structure 106 can also be folded and unfolded to allow frame 102 to deform during insertion into the aperture. A foldable and unfoldable plugging structure can be either elastic or non-elastic and may include a cloth or polymeric material. In one embodiment, plugging structure 106 is made of polyester.

Plugging structure 106 can include guide wire aperture 140 for insertion of a guide wire (not shown) during installation of the plug in a patient. Guide wire aperture 140 may be self-closing after the removal of a guide wire. In elastic embodiments, the self closing feature may be effected by the elasticity of plugging structure 106. In cloth embodiments, which may or may not be elastic, the woven fibers under tension from frame 102 can automatically close guide wire aperture 140.

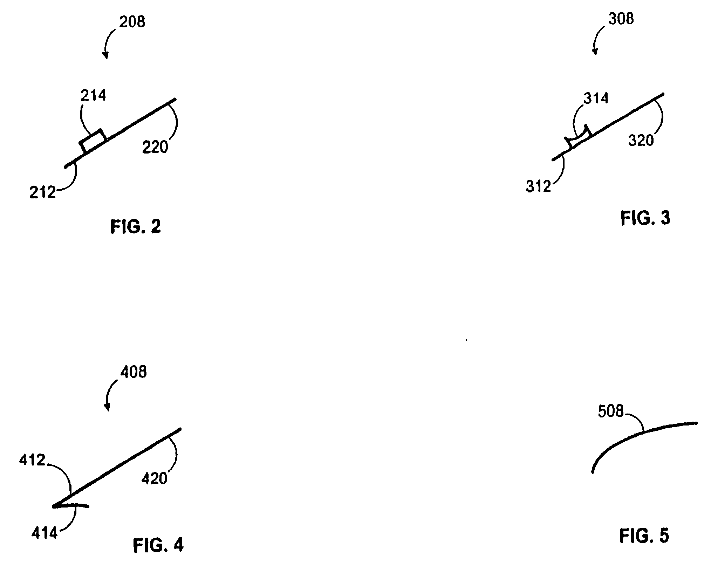

Remote ends 112 can also be provided with retention device receptacles for engaging a retention device that is part of a system for delivering the plug to an aperture in a wall. Finger aperture 114 can be used as a retention device receptacle. A nose cone cover (e.g., cover 314, shown in FIG. 3), is an alternative to a retention device receptacle. The delivery system is discussed below.

As shown in FIG. 1, fingers 108 and 110 can extend substantially radially away from central axis 104, even though some of those fingers may have tangential or spiral components and may not conform to a radial pattern. One or more of fingers 108 and 110 may also contain marker structures, such as a marker band. FIG. 1A shows a partial side view of plug 100 with fingers 108 and 110 in an intermediate configuration without plugging structure 106. For illustrative purposes, only a small number of fingers are shown in FIG. 1A. In an intermediate configuration, fingers are neither parallel nor perpendicular to central axis 104. As can be seen from FIG. 1A, cross-section 160, which is perpendicular to central axis 104 and passes through the medial section of plug 100, is discontinuous.

FIGS. 2-5 show different features that can be incorporated into a finger. FIG. 2, for example, shows a side view of finger 208, having proximal end 220 and remote end 212, with rivet 214 mounted thereto. Rivet 214 can be mounted on a finger such that the rivet head engages the patient's heart wall or such that it faces toward the heart cavity. FIG. 3 shows finger 308, having proximal end 320 and distal end 312, with nose cone cover 314. FIG. 4 shows finger 408, having proximal end 420 and distal end 412, with barb 414. FIG. 5 shows finger 508 which is curved. If a plug has two sets of fingers (as shown in FIG.1), the fingers of each set may be curved toward each other

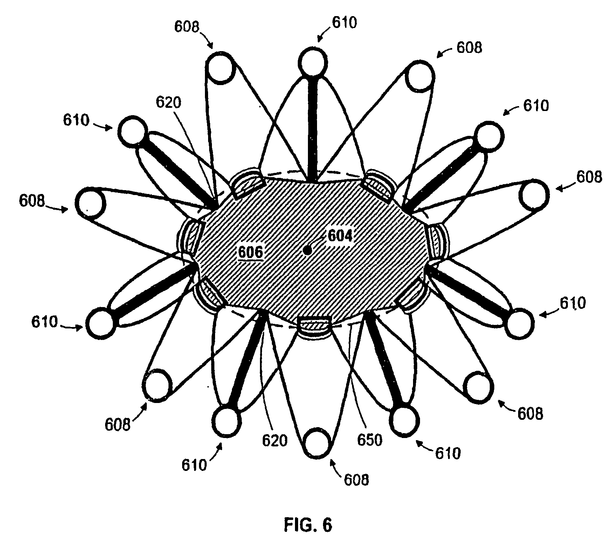

FIG. 6 shows another form of a plug constructed in accordance with this invention in which proximal ends 620 of fingers 608 and 610 define substantially elliptical cross section 650. Elliptical cross section 650 is in contrast to round cross-section 150 shown in FIG. 1. As shown in FIG. 6, central axis 604 passes near the center point of the ellipse. Plugging structure 606, which is supported by proximal ends 620, has a substantilly elliptical shape and could ave a guide wire aperture (not shown), if desired

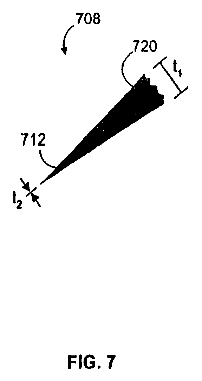

FIG. 7 shows an example of finger 708, which has proximal end 720 and distal end 712. Finger 708 tapers from thickness t₁ at proximal end 720 to lesser thickness t₂ at end 712 (e.g., remote from the plug's central axis). Conversely, finger 708 may be thicker at the remote end and thinner at the proximal end. Thus, a finger, such as finger 708, can have a resulting flexural stiffness that varies along its length. Fingers of varying width, such as the fingers shown in FIG. 1, can also have flexural stiffnesses that vary along their lengths regardless of variations in thickness by varying composition along the length.

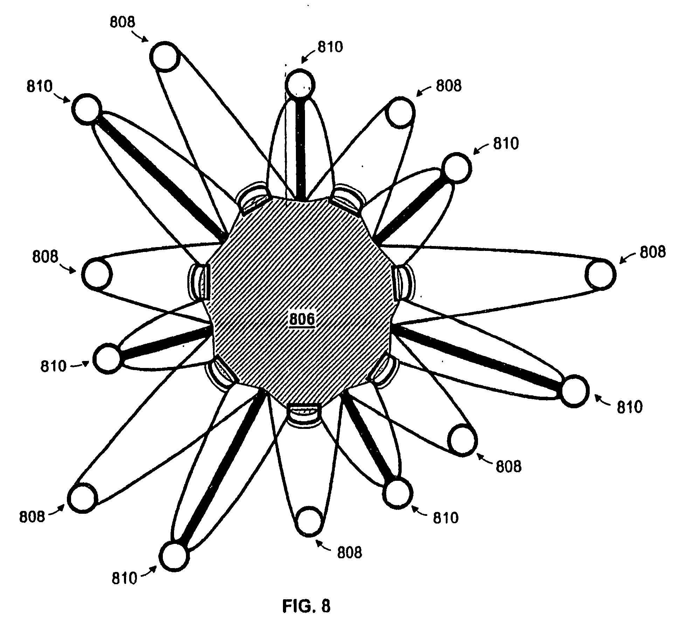

FIG. 8 shows another illustrative form of a plug in which fingers 808 and 810 have lengths that vary with respect to each other. Although fingers 808 and 810 have different lengths, it will be appreciated that plugging structure 806 can have a substantially circular, or any other convenient, shape.

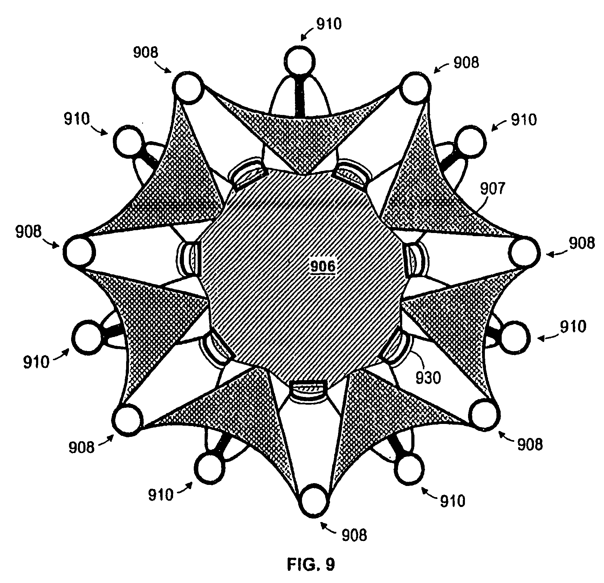

FIG. 9 shows another alternative form of a plug in which elastic web 907 spans between adjacent fingers of a plurality of fingers 908. The web can be made from any elastic material, including silicone. Plugging structure 906 is supported by proximal ends of fingers 908 and 910, and can be supported by support structures 930.

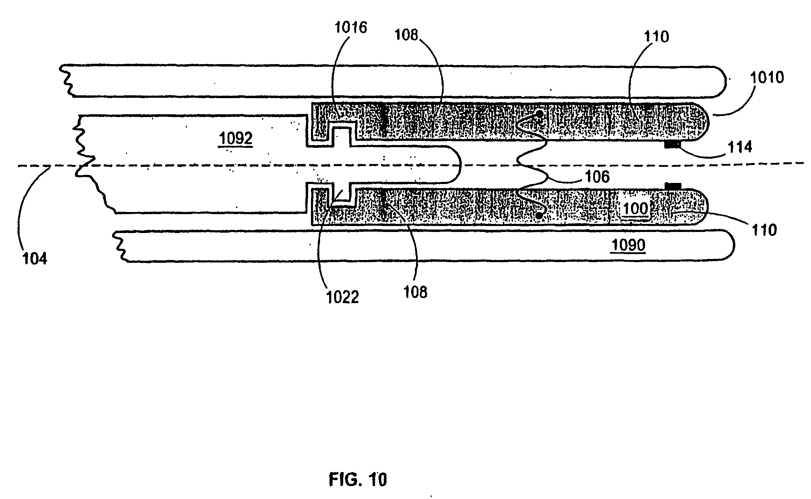

FIG. 10 shows how a plug, such as plug 100, can be installed via a delivery device, such as delivery catheter 1090. Plug 100 is inserted into delivery catheter 1090 by orienting fingers 108 and 110 in a direction that is substantially parallel to central axis 104. Next, plug 100 is passed through to distal opening 1010. Optional marker rivets 114 are shown in FIG. 10. Although it will be appreciated that plugging structure 1006 can be any convenient type, plugging structure 1006 is shown as a folded plugging structure. Retention device receptacles 1016 are engaged with locking pins 1022 of retention device 1092.

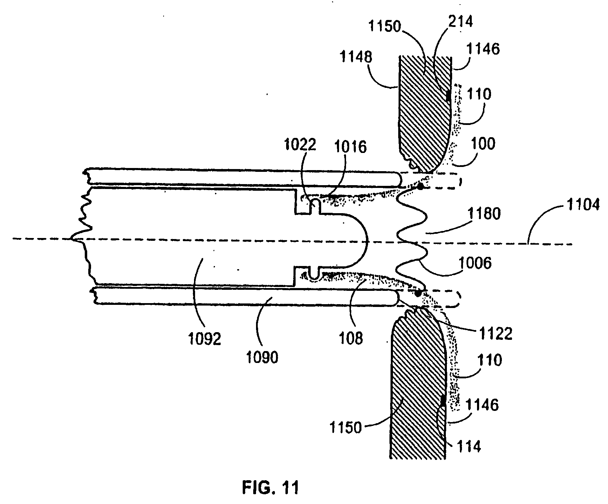

Once plug 100 is positioned near distal opening 1010, plug 100 can be inserted into aperture 1180 in wall 1150 of a patient's body cavity, as shown in FIG. 11. Initially, end 1122 of delivery catheter 1090 is positioned within aperture 1180 (indicated by a dashed line). Delivery catheter 1090 is then partially reciprocated away from aperture 1180 along axis 1104 and with respect to retention device 1092 (indicated by solid line). This forces fingers 110 out of delivery catheter 1090 and causes fingers 110 to spring out radially away from central axis 1104, thereby causing fingers 110 to engage side 1146 of wall 1150. At this stage, fingers 110 conform to the wall and perimeter of aperture 1180. Although FIG. 11 shows markers 114 attached to fingers 110 so they face wall 1150, it will be appreciated that these markers could also be located on the opposite side of these fingers

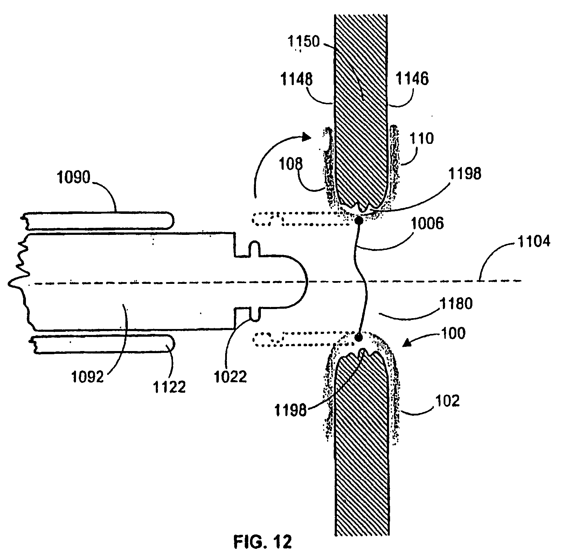

After catheter 1090 is partially reciprocated as shown in FIG. 11, catheter is further reciprocated as shown in FIG. 12. End 1122 is withdrawn past locking pins 1022 and retention device receptacles 1016. This allows fingers 108 to spring out radially away from central axis 1104 (from a position indicated by the dashed lines) and engage patient's cavity wall 1150 at surface 1148. As shown in FIG. 12, foldable plugging structure 1006 at least partially unfolds to span aperture 1180.

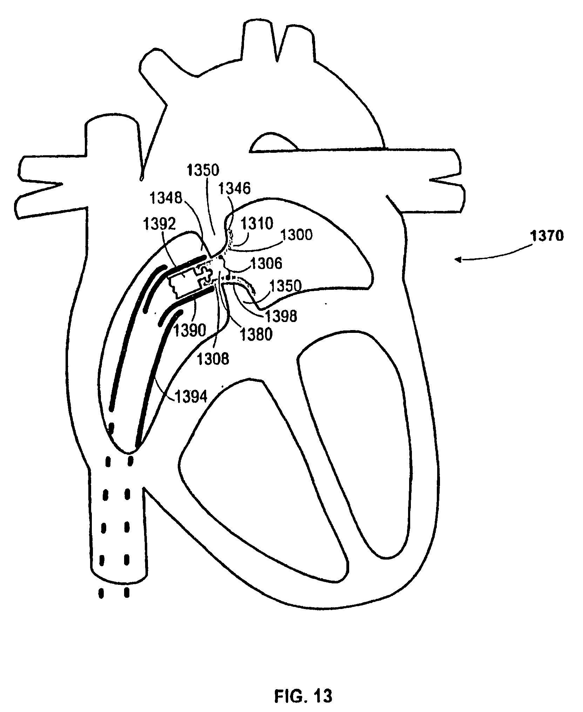

Figs shows plug 1300 partially installed in aperture 1380 in wall 1350 of heart 1370. Plug 1300 of FIG. 13 corresponds roughly to plug 100 of FIG. 11. Delivery catheter 1390 and retention device 1392 are guided to wall 1350 by delivery guide 1394. At this stage of the installation, fingers 1310 are deployed and engaged with surface 1346 of wall 1350. Fingers 1308 remain in delivery catheter 1390. Plugging structure 1306 is positioned inside aperture 1380 and is ready to conform to perimeter 1398 of aperture 1380 when the remainder of plug 1300 is released from retention device 1392

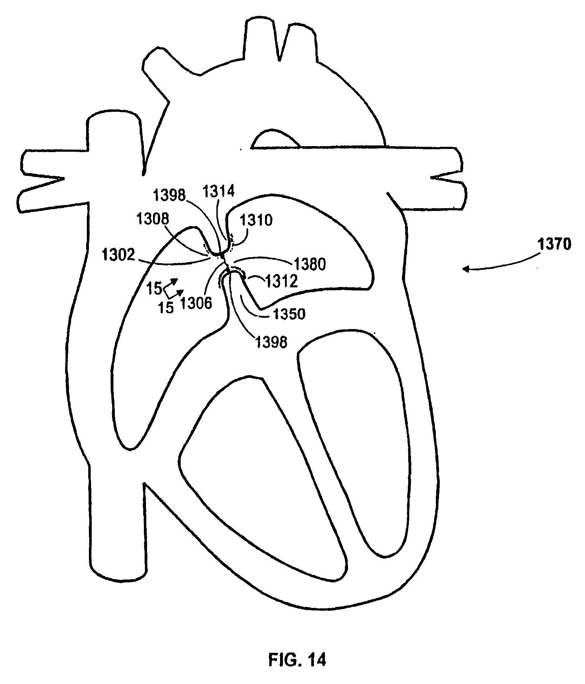

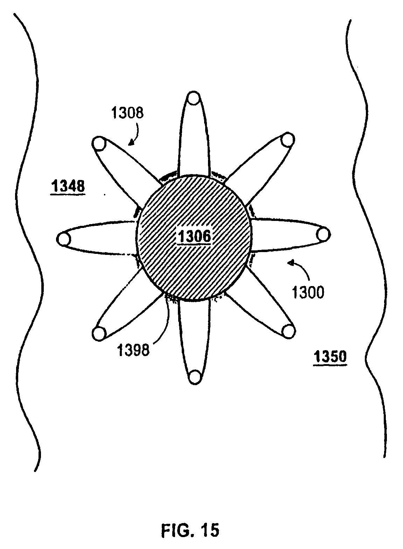

FIG. 14 shows plug 1300 fully installed in aperture 1380 in heart 1370 so that plugging structure 1306 spans aperture 1380 and frame 1302 conforms to perimeter 1398. Fingers 1308 and 1310 are engaged with opposite sides of wall 1350 to secure plug 1300. Optional markers 1314 are provided on ends 1312 of fingers 1310. In one form, markers 1314 are radiopaque. FIG. 15 shows plug 1300 as installed in wall 1350 as viewed along direction 15-15 of FIG. 14. Fingers 1308 are pressing against surface 1348 of wall 1350. Plugging structure 1306 spans the aperture in wall 1350 and is substantially flush with perimeter 1398

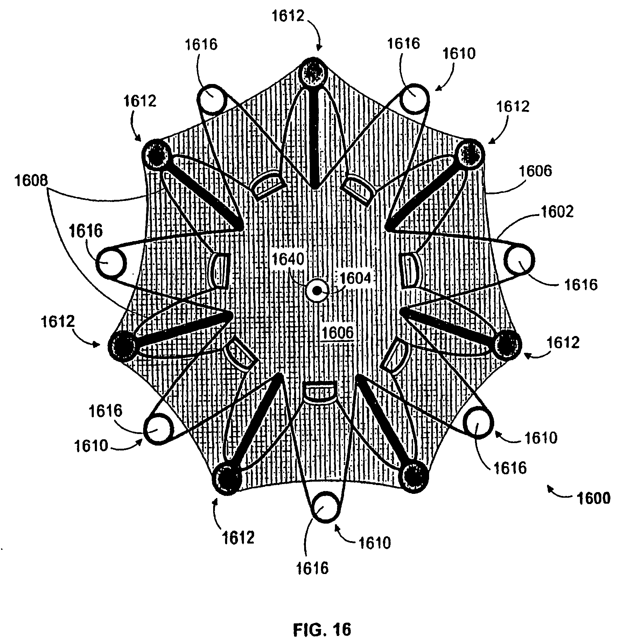

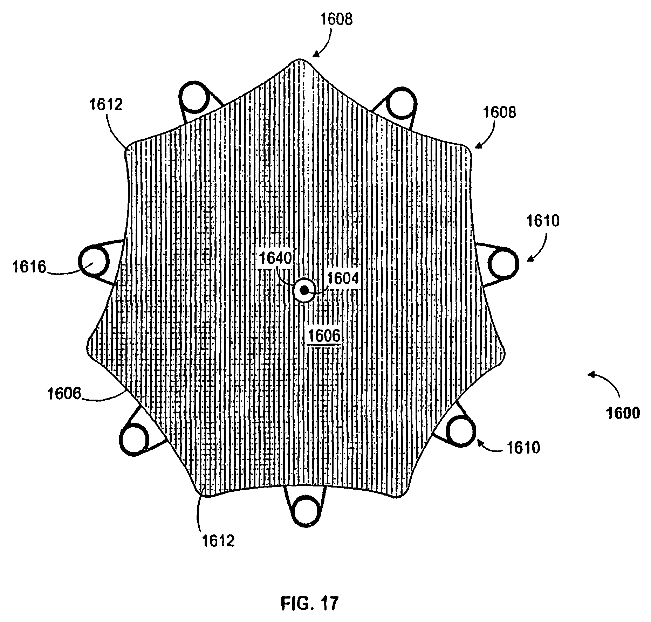

FIG. 16 shows another form of a plug. Plug 1600 includes frame 1602 which is structurally similar to frame 102 of FIG. 1. In this embodiment, however, plugging structure 1606 is supported by remote ends 1612 of fingers 1608. It will be appreciated that plugging structure could just as easily be mounted on fingers 1610. Optional marker rivets (not shown) can be placed in or near the apertures located at the of ends 1612 of fingers. Optional marker rivets can also be used to attach plugging structure 1606 to frame 1602 and simultaneously provide a radiopaque marking device for locating and positioning plug 1600 using medical scanning instrumentation. Central axis 1604 passes through plugging structure 1606. Guide wire aperture 1640 allows a guide wire to be used to help control the position of plug 1600 during installation. FIG. 17 shows plug 1600 as viewed from the side opposite that shown in FIG. 16. As shown in FIG. 17, fingers 1610 and retention device receptacles 1616 can radially extend beyond plugging structure 1606.

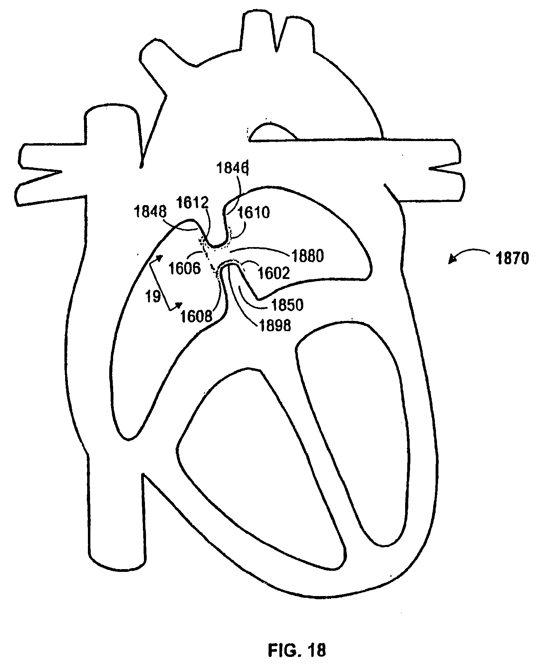

FIG. 18 shows plug 1600 fully installed in aperture 1880 of wall 1850 in patient's heart 1870. Plugging structure 1606 is attached to remote ends 1612 of fingers 1608 and is drawn against surface 1848 by fingers 1612 as fingers 1608 press against side 1848. When installed, plugging structure 1606 has a greater diameter than cavity wall aperture 1880 and thus extends beyond perimeter 1898 of aperture 1880 in order to occlude aperture 1880. Fingers 1610 engage side 1846 of wall 1850 and hold plug 1600 in position.

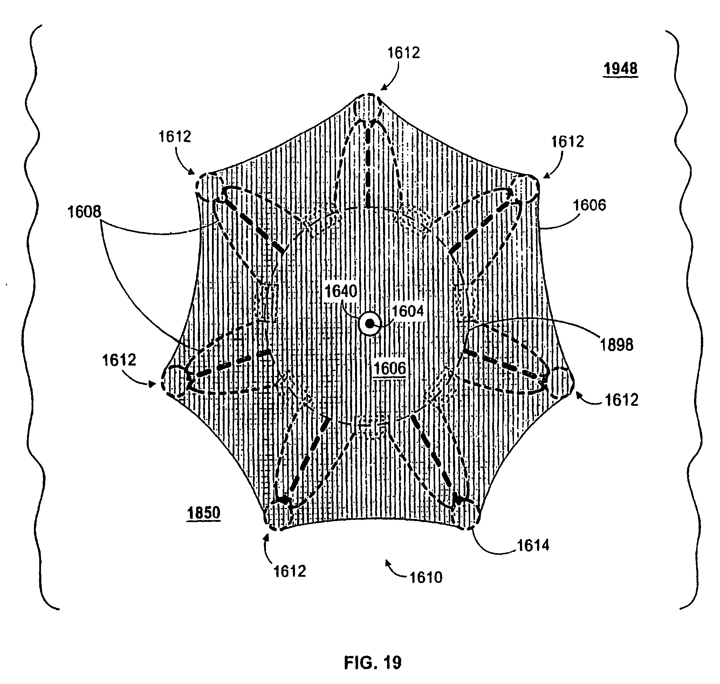

FIG. 19 shows plug 1600 as viewed from line 19-19 of FIG. 18. Fingers 1610 of frame 1602 are not shown because from this perspective they are behind wall 1850. Perimeter 1898 of aperture 1880 is shown as a broken line because it is behind plugging structure 1606. Plugging structure 1606 can be supported by rivets 1614, which are located at ends 1612.

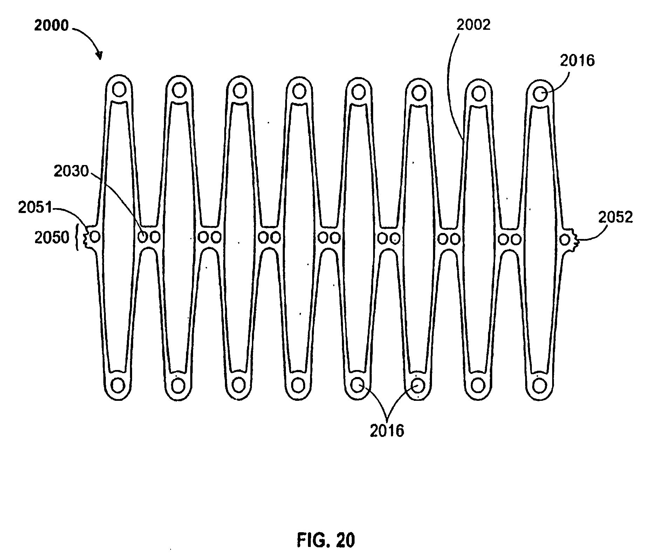

according to an embodiment of the invention, a plug has a medial portion that defines a central passage with a central axis. To allow the medial portion to expand radially, any cross-section of that portion (and preferably the entire frame) taken perpendicularly to the central axis is is discontinuous. FIG. 20 shows frame 2002 in an "unfurled" state. Portions 2051 and 2052 of medial section 2050 would normally be connected so that medial section 2050 forms a perforated tube surrounding the plug's central axis. Plug 2000 can include retention device receptacles 2016 on, for example, remote ends of the fingers. Frame 2002 can also include support structures 2030 for supporting a plugging structure (not shown).

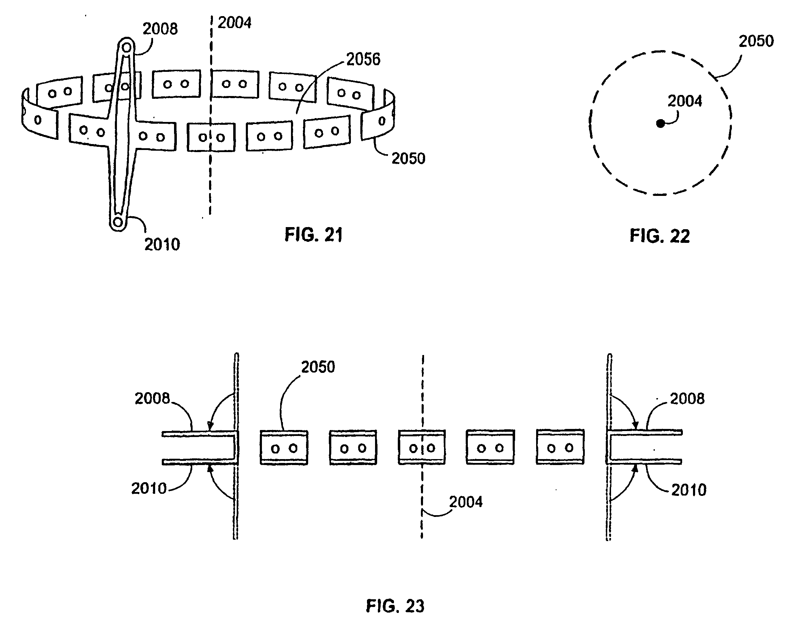

Medial section 2050, when in its operable shape forms a perforated tubular portion. FIG. 21 is a perspective view of frame 2002 in its operable tubular shape, including particularly medial section 2050 and fingers 2008 and 2010 (remainder of fingers not shown for the sake of simplicity). Perforated tubular portion 2050 defines longitudinal passage 2056 along central axis 2004.

FIG. 22 shows a cross-sectional view of perforated tubular portion 2050 taken along a plane that is perpendicular central axis 2004. The broken line corresponding to portion 2050 indicates that the cross section is discontinuous.

FIG. 23 shows a side view of tubular portion 2050 and selected fingers 2008 and 2010 in two different positions. As shown, fingers 2008 and 2010 are attached to the axial ends of tubular portion 2050 and are bent away from central axis 2004 into a played position. These fingers can be heat treated before the plug is installed to cause them to relax in this splayed position. Fingers 2008 and 2010 can also be positioned so that they point in a direction that is substantially parallel to central axis 2004 (as shown by dashed lines which corresponds to the view of FIG. 22) for installation of the plug. This position can be achieved, for example, by inserting the plug into a delivery device.

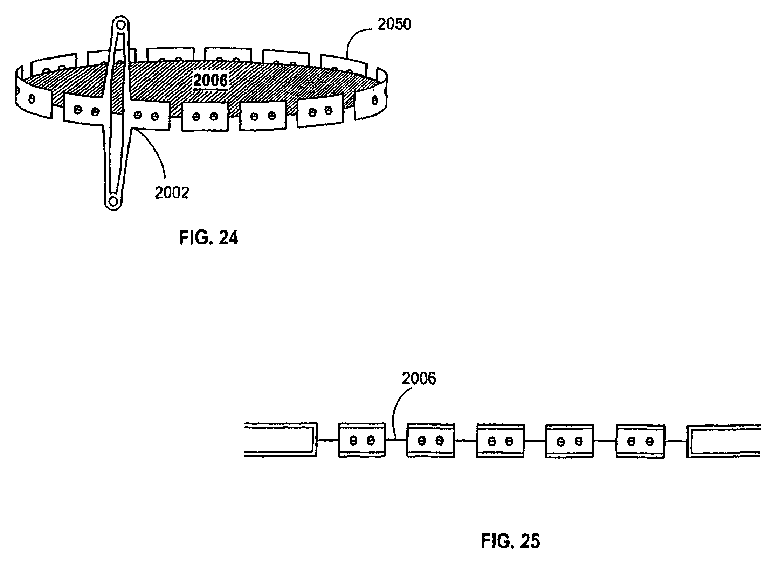

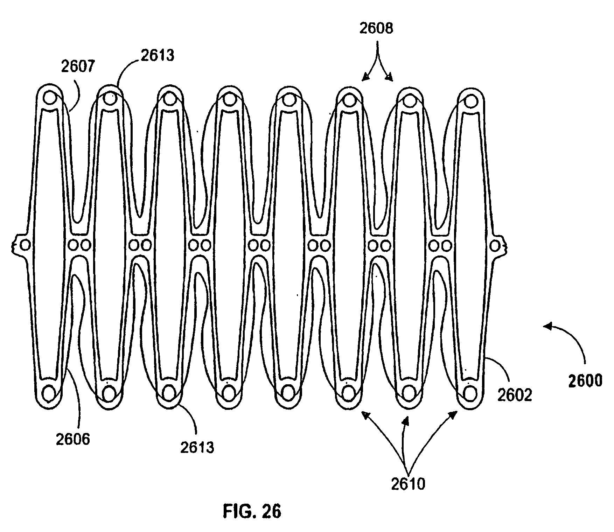

FIGS. 24 and 25 are similar to FIGS. 21 and 23, respectively, but now include plugging structure 2006 mounted to frame 2002. Alternatively, a plugging structure can be supported by support structures 2013 of fingers 2008 and/or 2010. Alternatively, plug 2000 can include two plugging structures, each of which can be supported by support structures located at opposite axial ends of plug 2000. For example, plug 2600, which includes frame 2602, is shown in FIG. 26 in an unfurled position for illustrative purposes. Frame 2602 includes first plugging structure 2606, which is mounted on support structures 2613 of fingers 2610 and second plugging structure 2607, which is mounted on support structures 2613 of fingers 2608. Support structures 2613 can also be used to mount markers or engage a retention device.

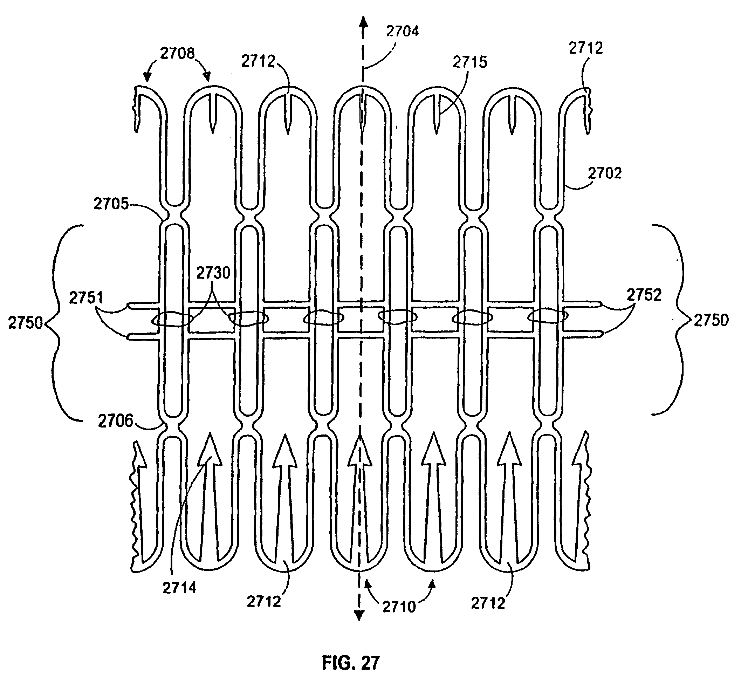

In another embodiment according to this invention, a frame having a perforated tubular portion can be formed from a plastically deformable material and can be installed using a balloon. FIG. 27, for example, shows frame 2702 in an unfurled state similar to the configuration of frame 2002 in FIGS. 20 and 26. In use, end portion 2751 and 2752 are joined to form a tubular structure surrounding central axis 2704. (Frame 2702 can be formed from a tube, thereby eliminating the need to join portions 2751 and 2752.)

When portions 2751 and 2752 are joined, medial section 2750 becomes a perforated tubular portion corresponding to perforated tubular portion 2050 shown in FIG. 22. Although the perforated tubular portion shown in FIG. 27 is axially longer than the perforated tubular portion shown in FIGS. 21 and 22, it will be appreciated that the length of the tubular portion can be matched to the thickness of the wall being plugged.

As shown in FIG. 27, fingers 2708 and fingers 2710 extend from opposite axial ends 2705 and 2706 of portion 2750, respectively. A plugging structure (not shown) can be attached, for example, to support structures 2730 to occlude the longitudinal passage during use. Piercing points 2715 and barbs 2714 are for engaging opposite sides of a wall for securing frame 2702 thereto.

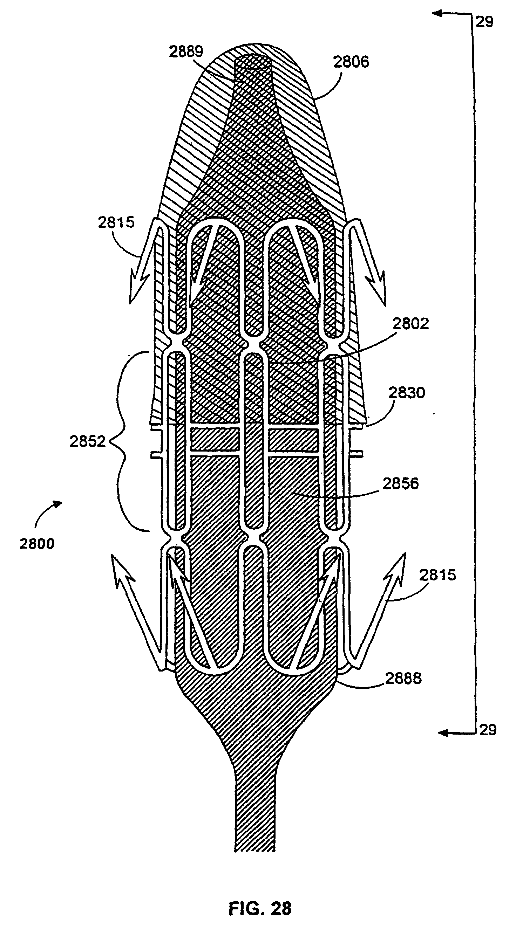

FIG. 28 shows plug 2800, which includes frame 2802 with delivery balloon 2888 inserted in longitudinal passage 2856. Delivery balloon 2888 is inflated enough to engage frame 2802 and allow delivery of plug 2800 to a repair site inside a patient. Folded or elastic plugging structure 2806 is deflected around tip 2889 of balloon 2888. Plugging structure 2806 is attached to frame 2802 at support structure 2830. Plug 2800 can have barbs 2815, for example, for engaging a patient's body tissue.

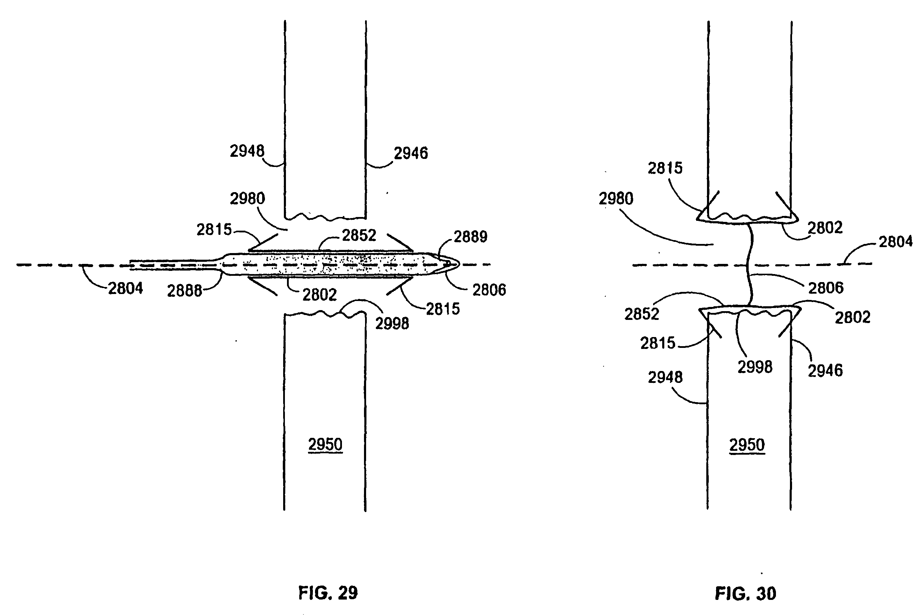

FIG. 29 shows a cross-sectional view of plug 2800 being positioned in aperture 2980 in cavity wall 2950. During installation of plug 2800, balloon 2888 engages the inside of frame 2802. Plugging structure 2806 is deflected around balloon tip 2889. Next, plug 2800 is inserted with balloon 2888 into aperture 2980. Balloon 2888 is then inflated, causing frame 2802 to expand radially and to contract along its central axis 2804, thereby forcing barbs 2815 to pierce opposite sides 2946 and 2948 of wall 2950. This also causes perforated tubular portion 2852 to conform to perimeter 2898. If plugging structure 2806 is secured to perforated tubular portion 2852, it too is stretched or unfolded across aperture 2980.

FIG. 30 shows plug 2800 installed in aperture 2980 after frame 2802 is deformed by the expansion of balloon 2888. At this stage, balloon 2888 has been removed from perforated tubular portion 2852. The perforations (i.e., the holes) in portion 2852 allow the axial length of perforated tubular portion 2852 to decrease as its radius increases.

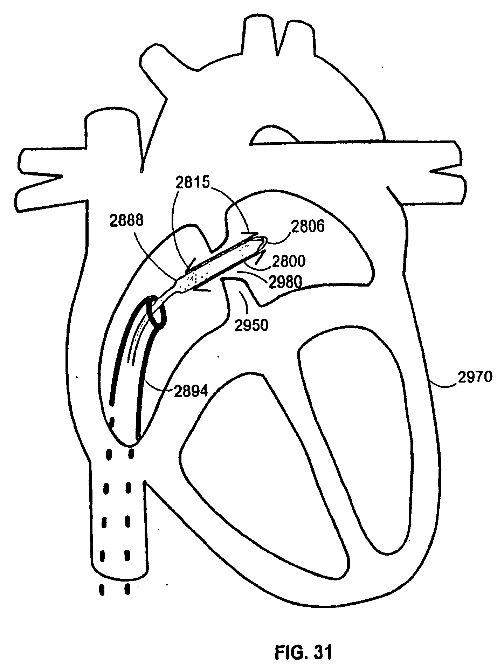

FIG. 31 shows plug 2800 being delivered to aperture 2980 in heart 2970. Plug 2800 is supported by partially inflated balloon 2888 and guided through a patient's body tissue and/or tubing via delivery guide 2894 until it is positioned with barbs 2815 on opposite sides of wall 2950.

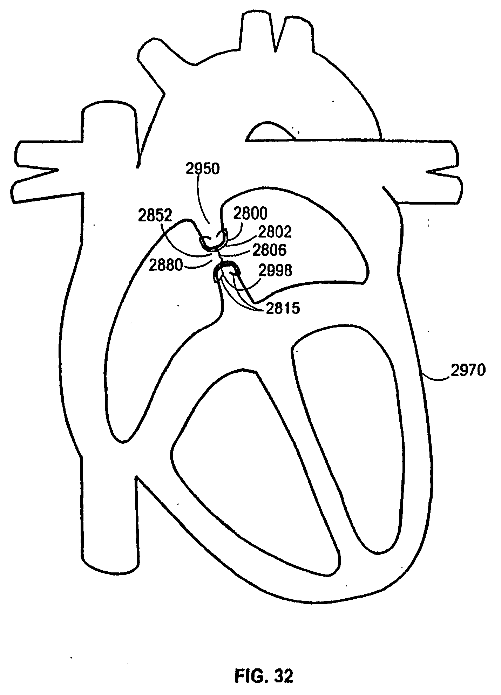

After delivery of plug 2800 to aperture 2980, plug 2800 can be fully installed by further expanding balloon 2888, thereby causing frame 2802 to deform and secure itself as shown in FIG. 32. After plug 2800 is secured, balloon 2888 is deflated and removed. When installed, tubular portion 2852 conforms to perimeter 2998 and plugging structure 2806 spans and occludes blood flow through the aperture.

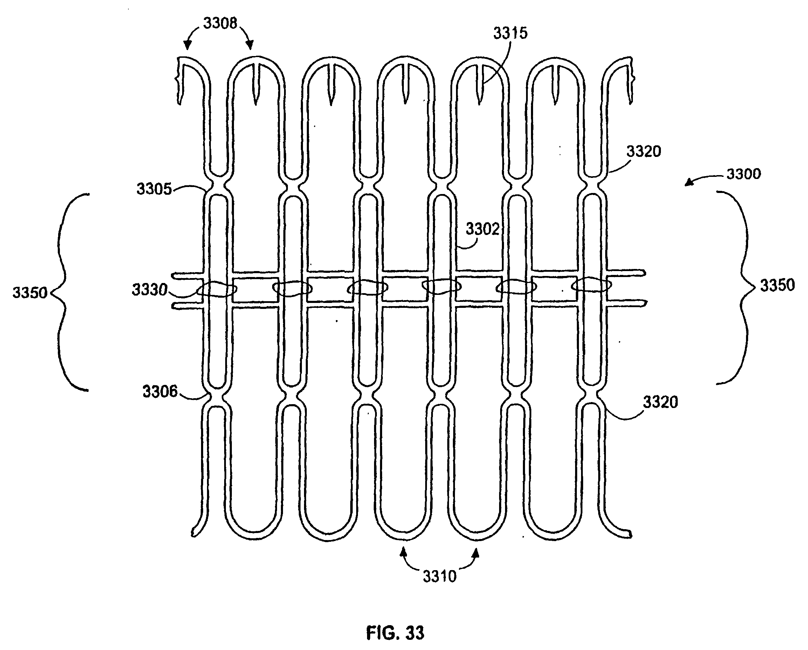

In another embodiment of the invention, a plug is provided for occluding a lumen of a patient's body tubing. FIG. 33 shows occlusion plug 3300, which includes frame 3302. Plug 3300 is similar aperture plug 2700 (shown in FIG. 27), but has barbs (or points) that extend from fingers 3308 on only one axial end 3305 of medial section 3350. It will be appreciated that points 3315 are not always necessary, but may be included in plugs where positive anchoring is desired. Frame 3302 is shown in FIG. 33 in an "unfurled" state, but as in the embodiments discussed above, would be joined to form a perforated tubular portion. Support structures 3330 can also be provided for securing a plugging structure (not shown).

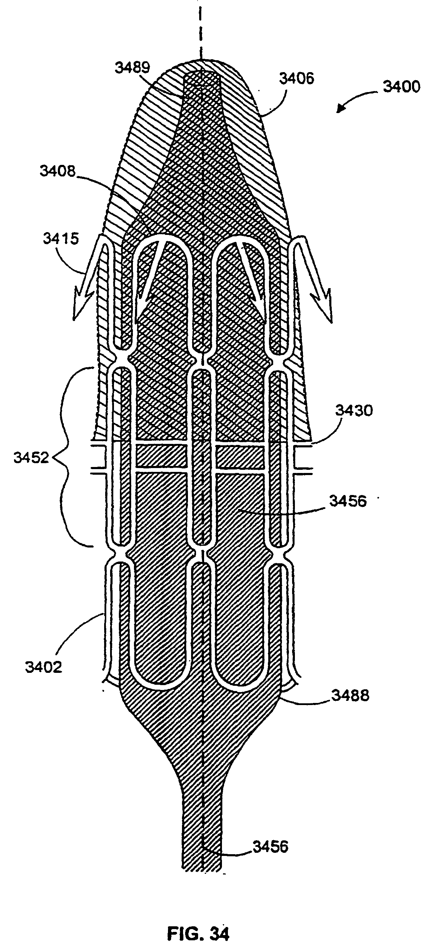

FIG. 34 shows occlusion plug 3400 mounted on partially inflated balloon 3488. Balloon 3488 occupies longitudinal passage along central axis 3456. Plugging structure 3406 is attached to frame 3402 at support structures 3430 in medial section 3452 and is deflected around tip 3489 of balloon 3488. Piercing points 3415, which can be barbed, extend away from central axis 3456 and are destined to be embedded in the interior wall of a patient's body tubing.

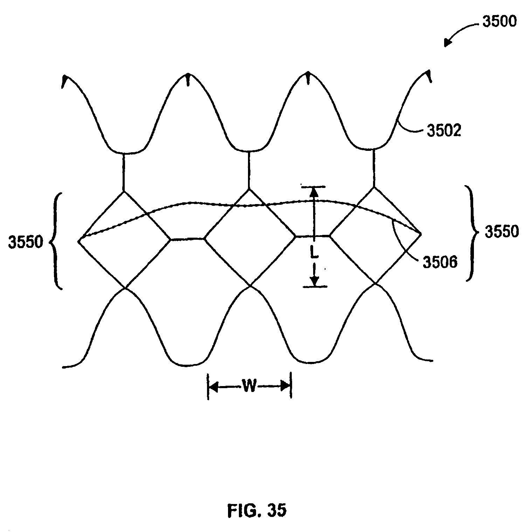

Occlusion plug 3500, which is shown in FIG. 35, is constructed according to this invention, but includes a frame with a different profile from that shown in FIG. 33. FIG. 35 shows a partial view of plug 3500 in the unfurled state. Like each of the other embodiments discussed above, medial portion 3550 can radially expand and axially contract when a balloon is inflated therein. In particular, each or the, rectangular units that make up frame 3502 can stretch, such that length L and width W vary inversely.

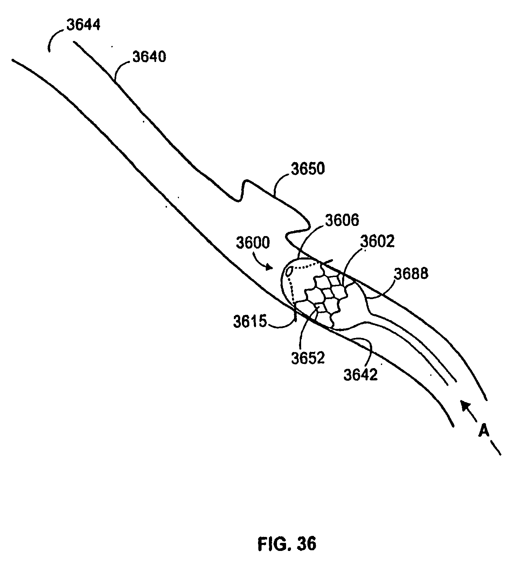

FIG. 36 shows the deployment of occlusion plug 3600 in a patient's blood vessel 3640 having aneurysm 3650. Arrow A shows the normal direction of blood flow through blood vessel 3640. Because of aneurysm 3650, it may be desirable to occlude blood vessel 3640 upstream from aneurysm 3650. As already explained above, with reference to plug 2800, for example, plug 3600 is mounted on balloon 3688 and positioned upstream of aneurysm 3650. As balloon 3688 is inflated, perforated tubular portion 3652 expands radially causing perforated tubular portion 3652 to conform to inner wall 3642 of blood vessel 3640. Plugging structure 3606 is attached to frame 3602 at points along the circumference of tubular portion 3652 and is thus stretched to occlude lumen 3644 of blood vessel 3640. As perforated tubular portion 3652 expands radially, it contracts axially and causes piercing points 3615 to engage walls 3642 and thus anchor plug 3600.

It will be understood that the foregoing is only illustrative of the principles of the invention, and that various modifications can be made by those skilled in the art without departing from the scope of the invention.

## Claims

1. A plug (2000, 2600) comprising:
a perforated tubular portion (2050) having a longitudinal passage (2056), wherein any cross-section of said perforated tubular portion (2050) in a plane perpendicular to said passage is discontinuous:
a plurality of finger (2008, 2010; 2608, 2610) attached to said tubular portion (2050) and extending from an axial end of said perforated tubular portion (2050) wherein any cross-section of said plurality of fingers (2008, 2010) in a plane perpendicular said passage is discontinuous; and
a plugging structure substantially occluding said passage.

2. The plug (2000, 2600) of claim 1 wherein said plug (2000) can be detected using fluoroscopy.

3. The plug (2000, 2600) of claim 1 wherein said perforated tubular portion (2050) and said plurality of fingers (2008, 2010) are formed from a unitary body.

4. The plug (2000, 2600) of claim 1 further comprising a second plurality of fingers (2010) attached to said tubular portion (2050) and extending from a second axial end of said perforated tubular portion.

5. The plug (2000, 2600) of claim 4 wherein said plug (2000, 2600) can be detected using fluoroscopy.

6. The plug (2000, 2600) of claim 4 wherein said perforated tubular portion (2050) and said first and second pluralities of fingers (2008, 2010; 2608, 2610) are formed from a unitary body

7. The plug (2000, 2600) of claim 4 wherein said perforated tubular structure (2050) and said fingers comprise an elastic material.

8. The plug (2000, 2600) of claim 7 wherein said perforated tubular structure (2050) and said fingers comprise nitinol.

9. The plug (2000, 2600) of claim 4 wherein at least one finger of said pluralities of fingers (2008, 2010; 2608, 2610) can be positioned to extend substantially radially away from said longitudinal passage.

10. The plug (2000, 2600) of claim 4 wherein at least one finger of said plurality of fingers (2608, 2610) has a marker structure.

11. The plug (2000, 2600) of claim 10 wherein said marker structure is radiopaque.

12. The plug (2000, 2600) of claim 10 wherein said marker structure is a marker band.

13. The plug (2000, 2600) of claim 12 wherein said finger has an end portion and said marker band is crimped to said end portion.

14. The plug (2000, 2600) of claim 10 wherein said marker structure is a rivet.

15. The plug (2000, 2600) of claim 4 wherein at least one finger of at least one of said pluralities of fingers has a retention device receptacle.

16. The plug (2000, 2600) of claim 15 wherein said retention device receptacle is a locking pin aperture.

17. The plug (2000, 2600) of claim 15 wherein said recension device receptacle is a nose cone cover.

18. The plug (2000, 2600) of claim 4 wherein at least one finger (2008, 2010) of at least one of said pluralities of fingers has a pointed end portion located remotely from a central axis (2004).

19. The plug (2000, 2600) of claim 4 wherein at least one finger of at least one of said pluralities of fingers has a barbed end portion located remotely from a central axis.

20. The plug (2000, 2600) of claim 4 wherein at least one finger (2008, 2010) of at least one of said pluralities of fingers is curved, said curve being concave toward a plane perpendicular to a central axis (2004) and passing substantially between said first and second pluralities of fingers.

21. The plug (2000, 2600) of claim 4 wherein said perforated tubular portion (2050) defines a substantially round cross section as viewed in a direction of said longitudinal axis.

22. The plug (2000, 2600) of claim 4 wherein said perforated tubular portion (2050) defines a substantially elliptical cross section as viewed in a direction of said longitudinal passage.

23. The plug (2000, 2600) of claim 4 wherein substantially all of said fingers (2008, 2010; 2608, 2610) of at least one of said pluralities are of substantially similar length.

24. The plug (2000, 2600) of claim 4 wherein different ones of the fingers (2000, 2010; 2608, 2610) of at least one of said pluralities are of different lengths.

25. The plug (2000, 2600) of claim 4 wherein at least one finger (2008, 2010; 2608, 2610) of at least one of said pluralities of fingers has different flexural stiffness along its length.

26. The plug (2000, 2600) of claim 4 wherein at least one finger (2008, 2010; 2608, 2610) of at least one of said pluralities of fingers has different thickness along its length.

27. The plug (2000, 2600) of claim 4 wherein at least one finger (2008, 2010; 2608, 2610) of at least one of said pluralities of fingers has different width along its length

28. The plug (2000, 2600) of claim 4 wherein at least one finger (2008, 2010; 2608, 2610) of at least one of said pluralities of fingers has a free end portion comprising an end structure configured to facilitate releasable retention of said finger by a plug delivery device.

29. The plug (2000, 2600) of claim 4 further comprising an elastic web between adjacent ones of said fingers (2008, 2010; 2608, 2610).

30. The plug (2000, 2600) of claim 29 wherein the web comprises silicone.

31. The plug (2000, 2600) of claim 4 wherein at least one finger (2008, 2010; 2608, 2610) of at least one of said pluralities of fingers has an end portion proximal to a central axis for supporting said plugging structure.

32. The plug (2000, 2600) of claim 4 wherein at least one finger (2008, 2010; 2608, 2610) of at least one of said pluralities of fingers has an end portion remote from a central axis for supporting said plugging structure.

33. The plug (2000, 2600) of claim 32 wherein said end portion has a supports structure (2613) with which to affix said plugging structure (2607).

34. The plug (2000, 2600) of claim 4 wherein said perforated tubular portion (2050) has at least one support structure with which to affix said plugging structure.

35. The plug (2000, 2600) of claim 4 wherein said plugging structure (2607, 2606) is elastic.

36. The plug (2000, 2600) of claim 4 wherein said plugging structure (2607, 2606) can be unfolded.

37. The plug (2000, 2600) of claim 4 wherein said plugging structure (2607, 2606) comprises polymeric material.

38. The plug (2000, 2600) of claim 4 wherein said plugging structure comprises cloth.

39. The plug (2000, 2600) of claim 4 wherein said plugging structure (2606) is sewn to a frame (2002).

40. The plug (2000, 2600) of claim 4 wherein said plugging structure (2606) has a guide wire aperture through which a guide wire can pass.

41. The plug (2000, 2600) of claim 40 wherein said guide wire aperture can substantially self close after said guide wire is removed.

42. The plug (2000, 2600) of claim 39 wherein said frame (2002) may be inserted into a delivery tube by extending said fingers in a direction substantially parallel to said longitudinal passage.

43. The plug (2000, 2600) of claim 4 wherein said perforated tubular portion (2050) can conform to a perimeter of a hole in a wall of a patient's body cavity.

44. The plug (2000, 2600) of claim 4 wherein said perforated tubular portion (2050) can contract longitudinally as it expands radially.

45. The plug (2000, 2600) of claim 4 wherein said perforated tubular portion (2050) is plastically deformable

46. The plug (2000, 2600) of claim 45 wherein said perforated tubular portion (2050) comprises stainless steel.

47. The plug (2000, 2600) of claim 46 wherein said tubular portion (2050) comprises tantalum.

48. The plug (2000, 2600) of claim 45 wherein said perforated tubular porrion (2050) can be conformed to a perimeter of a hole in a wall of a patient's body cavity using a balloon to expand said perforated tubular portion.

49. The plug (2000, 2600) of Claim 45 wherein said perforated tubular portion can contract longitudinally as it expands radially.

50. The plug (2000, 2600) of claim 1 wherein said perforated tubular portion (2050) and said fingers comprise an elastic material.

51. The plug (2000, 2600) of claim 50 wherein said perforated tubular portion and said fingers comprise nitinol.

52. The plug (2000, 2600) of claim 1 wherein at least one of said fingers (2008, 2010) extends substantially radially away from a central axis.

53. The plug (2000, 2600) of claim 1 wherein at least one of said fingers (2008, 2010; 2608, 2610) has a marker structure.

54. The plug (2000, 2600) of claim 53 wherein said marker structure is radiopaque.

55. The plug (2000, 2600) of claim 53 wherein said marker structure is a marker band.

56. The plug (2000, 2600) of claim 55 wherein said finger has an end portion and said marker band is crimped to said end portion.

57. The plug (2000, 2600) claim 53 wherein said marker structure is a rivet.

58. The plug (2000, 2600) of claim 1 wherein at least one of said fingers (2008, 2010; 2608, 2610) has a pointed end portion located remotely from a central axis.

59. The plug (2000, 2600) of claim 1 wherein at least one of said fingers has a barbed end portion located remotely from a central axis.

60. The plug (2000, 2600) of claim 1 wherein at least one of said fingers (2008, 2010; 2608, 2610) is curved, said curve being concave toward a plane perpendicular to a central axis and passing through said perforated tubular portion (2050).

61. The plug (2000, 2600) of claim 1 wherein said perforated tubular portion (2050) defines a substantially round cross section as viewed in a direction of said longitudinal passage.

62. The plug (2000, 2600) of claim 1 wherein said perforated tubular portion (2050) defines a substantially elliptical cross section as viewed in a direction of said longitudinal passage.

63. The plug (2000, 2600) of claim 1 wherein substantially all of said fingers (2008, 2010; 2608, 2610) are of substantially similar length.

64. The plug (2000, 2600) of claim 1 wherein different ones of the fingers (2008, 2010; 2608, 2610) are of different lengths.

65. The plug (2000, 2600) of claim 1 wherein at least one of said fingers (2008, 2010; 2608, 2610) has different flexural stiffness along its length.

66. The plug (2000, 2600) of claim 1 wherein at least one of said fingers (2008, 2010; 2608, 2610) has different thickness along its length.

67. The plug (2000, 2600) of claim 1 wherein at least one of said fingers (2008, 2010; 2608, 2610) has different width along its length.

68. The plug (2000, 2600) of claim 1 wherein at least one of said fingers (2008, 2010; 2608, 2610) has a free end portion comprising an end structure configured to facilitate releasable retention of said finger by a plug delivery device.

69. The plug (2000, 2600) of claim 1 further comprising an elastic web between adjacent ones of said fingers.

70. The plug (2000, 2600) of claim 69 wherein the web comprises silicone.

71. The plug (2000, 2600) of claim 1 wherein at least one of said fingers has an end portion proximal to a central axis for supporting said plugging structure.

72. The plug (2000, 2600) of claim 71 wherein said end portion has a support structure with which to affix said plugging structure.

73. The plug (2000, 2600) of claim 1 wherein said perforated tubular portion (2050) has at least one support structure for supporting said plugging structure.

74. The plug (2000, 2600) of claim 1 wherein said plugging structure is elastic.

75. The plug (2000, 2600) of claim 1 wherein said plugging structure can be unfolded.

76. The plug (2000, 2600) of claim 1 wherein said plugging structure comprises polymeric material.

77. The plug (2000, 2600) of claim 1 whereon said plugging structure comprises cloth.

78. The plug (2000, 2600) of claim 1 wherein said plugging structure (2006) is sewn to a frame (2002).

79. The plug (2000, 2600) of claim 1 wherein said plugging structure (2606, 2607) has a guide wire aperture through which a guide wire can pass.

80. The plug (2000, 2600) of claim 79 wherein said guide wire aperture can substantially self close after said guide wire is removed.

81. The plug (2000, 2600) of claim 1 wherein a frame (2002) may be inserted into a delivery tube by extending said fingers in a direction substantially parallel to said longitudinal passage.

82. The plug (2000, 2600) of claim 1 wherein said perforated tubular portion (2050) can conform to a perimeter of a defect in a wall of a patient's body cavity.

83. The plug (2000, 2600) of claim 1 wherein said perforated tubular portion (2050) can contract longitudinally as it expands radially.

84. The plug (2000, 2606) of claim 1 wherein said perforated tubular portion (2050) is plastically deformable.

85. The plug (2000, 2600) of claim 84 wherein said perforated tubular portion (2050) comprises stainless steel.

86. The plug (2000, 2600) of claim 84 wherein said perforated tubular portion (2050) comprises tantalum.

87. The plug (2000, 2600) of claim 84 wherein said perforated tubular portion (2050) can be conformed to a perimeter of a hole in a wall of a patient's body cavity using a balloon to expand said perforated tubular portion.

88. The plug (2000, 2600) of claim 84 wherein said perforated tubular portion (2050) can contract longitudinally as it expands radially.

## Patentansprüche

1. Stopfen (2000, 2600) mit:
einem perforierten Röhrenabschnitt (2050) mit einem Längsdurchgang (2056), wobei jeder Querschnitt des perforierten Röhrenabschnitts (2050) in einer Ebene senkrecht zum Durchgang diskontinuierlich ist;
mehreren Fingern (2008, 2010; 2608, 2610), die am Röhrenabschnitt (2050) befestigt sind und sich von einem Axialende des perforierten Röhrenabschnitts (2050) erstrecken, wobei jeder Querschnitt der mehreren Finger (2008, 2010) in einer Ebene senkrecht zum Durchgang diskontinuierlich ist; und
einer Verschlußstruktur, die den Durchgang im wesentlichen okkludiert.

2. Stopfen (2000, 2600) nach Anspruch 1, wobei der Stopfen (2000) mit Hilfe von Fluoroskopie detektiert werden kann.

3. Stopfen (2000, 2600) nach Anspruch 1, wobei der perforierte Röhrenabschnitt (2050) und die mehreren Finger (2008, 2010) aus einem einteiligen Körper gebildet sind.

4. Stopfen (2000, 2600) nach Anspruch 1, ferner mit einem zweiten Satz von Fingern (2010), die am Röhrenabschnitt (2050) befestigt sind und sich von einem zweiten Axialende des perforierten Röhrenabschnitts erstrecken.

5. Stopfen (2000, 2600) nach Anspruch 4, wobei der Stopfen (2000, 2600) mit Hilfe von Fluoroskopie detektiert werden kann.

6. Stopfen (2000, 2600) nach Anspruch 4, wobei der perforierte Röhrenabschnitt (2050) sowie der erste und zweite Satz von Fingern (2008, 2010; 2608, 2610) aus einem einteiligen Körper gebildet sind.

7. Stopfen (2000, 2600) nach Anspruch 4, wobei der perforierte Röhrenabschnitt (2050) und die Finger ein elastisches Material aufweisen.

8. Stopfen (2000, 2600) nach Anspruch 7, wobei der perforierte Röhrenabschnitt (2050) und die Finger Nitinol aufweisen.

9. Stopfen (2000, 2600) nach Anspruch 4, wobei mindestens ein Finger der Sätze von Fingern (2008, 2010; 2608, 2610) so positioniert sein kann, daß er sich vom Längsdurchgang im wesentlichen radial weg erstreckt.

10. Stopfen (2000, 2600) nach Anspruch 4, wobei mindestens ein Finger der Sätze von Fingern (2608, 2610) eine Markerstruktur hat.

11. Stopfen (2000, 2600) nach Anspruch 10, wobei die Markerstruktur röntgendicht ist.

12. Stopfen (2000, 2600) nach Anspruch 10, wobei die Markerstruktur ein Markerband ist.

13. Stopfen (2000, 2600) nach Anspruch 12, wobei der Finger einen Endabschnitt hat und das Markerband auf den Endabschnitt aufgecrimpt ist.

14. Stopfen (2000, 2600) nach Anspruch 10, wobei die Markerstruktur ein Niet ist.

15. Stopfen (2000, 2600) nach Anspruch 4, wobei mindestens ein Finger mindestens eines der Sätze von Fingern eine Haltevorrichtungsaufnahme hat.

16. Stopfen (2000, 2600) nach Anspruch 15, wobei die Haltevorrichtungsaufnahme eine Arretierstiftöffnung ist.

17. Stopfen (2000, 2600) nach Anspruch 15, wobei die Haltevorrichtungsaufnahme eine Nasenkonusabdeckung ist.

18. Stopfen (2000, 2600) nach Anspruch 4, wobei mindestens ein Finger (2008, 2010) mindestens eines der Sätze von Fingern einen spitzen Endabschnitt hat, der von einer Mittelachse (2004) entfernt liegt.

19. Stopfen (2000, 2600) nach Anspruch 4, wobei mindestens ein Finger mindestens eines der Sätze von Fingern einen widerhakenförmigen Endabschnitt hat, der von einer Mittelachse entfernt liegt.

20. Stopfen (2000, 2600) nach Anspruch 4, wobei mindestens ein Finger (2008, 2010) mindestens eines der Sätze von Fingern gekrümmt ist, wobei die Krümmung konkav zu einer Ebene senkrecht zu einer Mittelachse (2004) ist und im wesentlichen zwischen dem ersten und zweiten Satz von Fingern verläuft.

21. Stopfen (2000, 2600) nach Anspruch 4, wobei der perforierte Röhrenabschnitt (2050) einen im wesentlichen runden Querschnitt im Blick in Richtung des Längsdurchgangs bildet.

22. Stopfen (2000, 2600) nach Anspruch 4, wobei der perforierte Röhrenabschnitt (2050) einen im wesentlichen elliptischen Querschnitt im Blick in Richtung des Längsdurchgangs bildet.

23. Stopfen (2000, 2600) nach Anspruch 4, wobei im wesentlichen alle Finger (2008, 2010; 2608, 2610) mindestens eines der Sätze im wesentlichen eine ähnliche Länge haben.

24. Stopfen (2000, 2600) nach Anspruch 4, wobei unterschiedliche der Finger (2008, 2010; 2608, 2610) mindestens eines der Sätze unterschiedliche Längen haben.

25. Stopfen (2000, 2600) nach Anspruch 4, wobei mindestens ein Finger (2008, 2010; 2608, 2610) mindestens eines der Sätze von Fingern eine unterschiedliche Biegesteifigkeit über seine Länge hat.

26. Stopfen (2000, 2600) nach Anspruch 4, wobei mindestens ein Finger (2008, 2010; 2608, 2610) mindestens eines der Sätze von Fingern eine unterschiedliche Dicke über seine Länge hat.

27. Stopfen (2000, 2600) nach Anspruch 4, wobei mindestens ein Finger (2008, 2010; 2608, 2610) mindestens eines der Sätze von Fingern eine unterschiedliche Breite über seine Länge hat.

28. Stopfen (2000, 2600) nach Anspruch 4, wobei mindestens ein Finger (2008, 2010; 2608, 2610) mindestens eines der Sätze von Fingern einen freien Endabschnitt mit einer Endstruktur hat, die so konfiguriert ist, daß sie lösbares Halten des Fingers durch eine Stopfenabgabevorrichtung erleichtert.

29. Stopfen (2000, 2600) nach Anspruch 4, ferner mit einer elastischen Bahn zwischen benachbarten der Finger (2008, 2010; 2608, 2610).

30. Stopfen (2000, 2600) nach Anspruch 29, wobei die Bahn Silikon aufweist.

31. Stopfen (2000, 2600) nach Anspruch 4, wobei mindestens ein Finger (2008, 2010; 2608, 2610) mindestens eines der Sätze von Fingern einen Endabschnitt proximal zu einer Mittelachse zum Abstützen der Verschlußstruktur hat.

32. Stopfen (2000, 2600) nach Anspruch 4, wobei mindestens ein Finger (2008, 2010; 2608, 2610) mindestens eines der Sätze von Fingern einen Endabschnitt entfernt von einer Mittelachse zum Abstützen der Verschlußstruktur hat.

33. Stopfen (2000, 2600) nach Anspruch 32, wobei der Endabschnitt eine Stützstruktur (2613) hat, mit der die Verschlußstruktur (2607) zu fixieren ist.

34. Stopfen (2000, 2600) nach Anspruch 4, wobei der perforierte Röhrenabschnitt (2050) mindestens eine Stützstruktur hat, mit der die Verschlußstruktur zu fixieren ist.

35. Stopfen (2000, 2600) nach Anspruch 4, wobei die Verschlußstruktur (2607, 2606) elastisch ist.

36. Stopfen (2000, 2600) nach Anspruch 4, wobei die Verschlußstruktur (2607, 2606) entfaltet werden kann.

37. Stopfen (2000, 2600) nach Anspruch 4, wobei die Verschlußstruktur (2607, 2606) Polymermaterial aufweist.

38. Stopfen (2000, 2600) nach Anspruch 4, wobei die Verschlußstruktur Gewebe aufweist.

39. Stopfen (2000, 2600) nach Anspruch 4, wobei die Verschlußstruktur (2606) mit einem Rahmen (2002) vernäht ist.

40. Stopfen (2000, 2600) nach Anspruch 4, wobei die Verschlußstruktur (2606) eine Führungsdrahtöffnung hat, die von einem Führungsdraht durchlaufen werden kann.

41. Stopfen (2000, 2600) nach Anspruch 40, wobei sich die Führungsdrahtöffnung im wesentlichen selbst verschließen kann, wenn der Führungsdraht entfernt ist.

42. Stopfen (2000, 2600) nach Anspruch 39, wobei der Rahmen (2002) in eine Abgaberöhre eingeführt werden kann, indem die Finger in eine Richtung im wesentlichen parallel zum Längsdurchgang orientiert werden.

43. Stopfen (2000, 2600) nach Anspruch 4, wobei sich der perforierte Röhrenabschnitt (2050) an einen Umfang eines Lochs- in einer Wand einer Körperhöhle eines Patienten anpassen kann.

44. Stopfen (2000, 2600) nach Anspruch 4, wobei der perforierte Röhrenabschnitt (2050) längs kontrahieren kann, wenn er radial expandiert.

45. Stopfen (2000, 2600) nach Anspruch 4, wobei der perforierte Röhrenabschnitt (2050) plastisch verformbar ist.

46. Stopfen (2000, 2600) nach Anspruch 45, wobei der perforierte Röhrenabschnitt (2050) Edelstahl aufweist.

47. Stopfen (2000, 2600) nach Anspruch 46, wobei der perforierte Röhrenabschnitt (2050) Tantal aufweist.

48. Stopfen (2000, 2600) nach Anspruch 45, wobei der perforierte Röhrenabschnitt (2050) an einen Umfang eines Lochs in einer Wand einer Körperhöhle eines Patienten mit Hilfe eines Ballons angepaßt werden kann, um den perforierten Röhrenabschnitt zu expandieren.

49. Stopfen (2000, 2600) nach Anspruch 45, wobei der perforierte Röhrenabschnitt längs kontrahieren kann, wenn er radial expandiert.

50. Stopfen (2000, 2600) nach Anspruch 1, wobei der perforierte Röhrenabschnitt (2050) und die Finger ein elastisches Material aufweisen.

51. Stopfen (2000, 2600) nach Anspruch 50, wobei der perforierte Röhrenabschnitt und die Finger Nitinol aufweisen.

52. Stopfen (2000, 2600) nach Anspruch 1, wobei sich mindestens einer der Finger (2008, 2010) im wesentlichen radial von einer Mittelachse weg erstreckt.

53. Stopfen (2000, 2600) nach Anspruch 1, wobei mindestens einer der Finger (2008, 2010; 2608, 2610) eine Markerstruktur hat.

54. Stopfen (2000, 2600) nach Anspruch 53, wobei die Markerstruktur röntgendicht ist.

55. Stopfen (2000, 2600) nach Anspruch 53, wobei die Markerstruktur ein Markerband ist.

56. Stopfen (2000, 2600) nach Anspruch 55, wobei der Finger einen Endabschnitt hat und das Markerband auf den Endabschnitt aufgecrimpt ist.

57. Stopfen (2000, 2600) nach Anspruch 53, wobei die Markerstruktur ein Niet ist.

58. Stopfen (2000, 2600) nach Anspruch 1, wobei mindestens einer der Finger (2008, 2010; 2608, 2610) einen spitzen Endabschnitt hat, der von einer Mittelachse entfernt liegt.

59. Stopfen (2000, 2600) nach Anspruch 1, wobei mindestens einer der Finger einen widerhakenförmigen Endabschnitt hat, der von einer Mittelachse entfernt liegt.

60. Stopfen (2000, 2600) nach Anspruch 1, wobei mindestens einer der Finger (2008, 2010; 2608, 2610) gekrümmt ist, wobei die Krümmung konkav zu einer Ebene senkrecht zu einer Mittelachse ist und durch den perforierten Röhrenabschnitt (2050) verläuft.

61. Stopfen (2000, 2600) nach Anspruch 1, wobei der perforierte Röhrenabschnitt (2050) einen im wesentlichen runden Querschnitt im Blick in Richtung des Längsdurchgangs bildet.

62. Stopfen (2000, 2600) nach Anspruch 1, wobei der perforierte Röhrenabschnitt (2050) einen im wesentlichen elliptischen Querschnitt im Blick in Richtung des Längsdurchgangs bildet.

63. Stopfen (2000, 2600) nach Anspruch 1, wobei im wesentlichen alle Finger (2008, 2010; 2608, 2610) im wesentlichen eine ähnliche Länge haben.

64. Stopfen (2000, 2600) nach Anspruch 1, wobei unterschiedliche der Finger (2008, 2010; 2608, 2610) unterschiedliche Längen haben.

65. Stopfen (2000, 2600) nach Anspruch 1, wobei mindestens einer der Finger (2008, 2010; 2608, 2610) eine unterschiedliche Biegesteifigkeit über seine Länge hat.

66. Stopfen (2000, 2600) nach Anspruch 1, wobei mindestens einer der Finger (2008, 2010; 2608, 2610) eine unterschiedliche Dicke über seine Länge hat.

67. Stopfen (2000, 2600) nach Anspruch 1, wobei mindestens einer der Finger (2008, 2010; 2608, 2610) eine unterschiedliche Breite über seine Länge hat.

68. Stopfen (2000, 2600) nach Anspruch 1, wobei mindestens einer der Finger (2008, 2010; 2608, 2610) einen freien Endabschnitt mit einer Endstruktur hat, die so konfiguriert ist, daß sie lösbares Halten des Fingers durch eine Stopfenabgabevorrichtung erleichtert.

69. Stopfen (2000, 2600) nach Anspruch 1, ferner mit einer elastischen Bahn zwischen benachbarten der Finger.

70. Stopfen (2000, 2600) nach Anspruch 69, wobei die Bahn Silikon aufweist.

71. Stopfen (2000, 2600) nach Anspruch 1, wobei mindestens einer der Finger einen Endabschnitt proximal zu einer Mittelachse zum Abstützen der Verschlußstruktur hat.

72. Stopfen (2000, 2600) nach Anspruch 71, wobei der Endabschnitt eine Stützstruktur hat, mit der die Verschlußstruktur zu fixieren ist.

73. Stopfen (2000, 2600) nach Anspruch 1, wobei der perforierte Röhrenabschnitt (2050) mindestens eine Stützstruktur zum Stützen der Verschlußstruktur hat.

74. Stopfen (2000, 2600) nach Anspruch 1, wobei die Verschlußstruktur elastisch ist.

75. Stopfen (2000, 2600) nach Anspruch 1, wobei die Verschlußstruktur entfaltet werden kann.

76. Stopfen (2000, 2600) nach Anspruch 1, wobei die Verschlußstruktur Polymermaterial aufweist.

77. Stopfen (2000, 2600) nach Anspruch 1, wobei die Verschlußstruktur Gewebe aufweist.

78. Stopfen (2000, 2600) nach Anspruch 1, wobei die Verschlußstruktur (2606) mit einem Rahmen (2002) vernäht ist.

79. Stopfen (2000, 2600) nach Anspruch 1, wobei die Verschlußstruktur (2606, 2607) eine Führungsdrahtöffnung hat, die von einem Führungsdraht durchlaufen werden kann.

80. Stopfen (2000, 2600) nach Anspruch 79, wobei sich die Führungsdrahtöffnung im wesentlichen selbst verschließen kann, wenn der Führungsdraht entfernt ist.

81. Stopfen (2000, 2600) nach Anspruch 1, wobei ein Rahmen (2002) in eine Abgaberöhre eingeführt werden kann, indem die Finger in eine Richtung im wesentlichen parallel zum Längsdurchgang orientiert werden.

82. Stopfen (2000, 2600) nach Anspruch 1, wobei sich der perforierte Röhrenabschnitt (2050) an einen Umfang eines Defekts in einer Wand einer Körperhöhle eines Patienten anpassen kann.

83. Stopfen (2000, 2600) nach Anspruch 1, wobei der perforierte Röhrenabschnitt (2050) längs kontrahieren kann, wenn er radial expandiert.

84. Stopfen (2000, 2600) nach Anspruch 1, wobei der perforierte Röhrenabschnitt (2050) plastisch verformbar ist.

85. Stopfen (2000, 2600) nach Anspruch 84, wobei der perforierte Röhrenabschnitt (2050) Edelstahl aufweist.

86. Stopfen (2000, 2600) nach Anspruch 84, wobei der perforierte Röhrenabschnitt (2050) Tantal aufweist.

87. Stopfen (2000, 2600) nach Anspruch 84, wobei der perforierte Röhrenabschnitt (2050) an einen Umfang eines Lochs in einer Wand einer Körperhöhle eines Patienten mit Hilfe eines Ballons angepaßt werden kann, um den perforierten Röhrenabschnitt zu expandieren.

88. Stopfen (2000, 2600) nach Anspruch 84, wobei der perforierte Röhrenabschnitt (2050) längs kontrahieren kann, wenn er radial expandiert.

## Revendications

1. Un bouchon (2000, 2600), comprenant :
une partie tubulaire (2050) perforée, ayant un passage longitudinal (2056), dans lequel toute section transversale de la partie tubulaire (2050) perforée, dans un plan perpendiculaire audit passage, est discontinue :
une pluralité de doigts (2008, 2010 ; 2608, 2610), attachés à ladite partie tubulaire (2050) et s'étendant depuis une extrémité axiale de ladite partie tubulaire (2050) perforée, dans lequel toute section transversale de ladite pluralité de doigts (2008, 2010), dans un plan perpendiculaire audit passage, est discontinue ; et
une structure de bouchage, obstruant sensiblement ledit passage.

2. Le bouchon (2000, 2600) selon la revendication 1, dans lequel ledit bouchon (2000) peut être détecté en utilisant une fluoroscopie.

3. Le bouchon (2000, 2600) selon la revendication 1, dans lequel ladite partie tubulaire (2050) perforée et ladite pluralité de doigts (2008, 2010) sont formés à partir d'un corps unitaire.

4. Le bouchon (2000, 2600) selon la revendication 1, comprenant en outre une deuxième pluralité de doigts (2010), attachés à ladite partie tubulaire (2050) et s'étendant depuis une deuxième extrémité axiale de ladite partie tubulaire perforée.

5. Le bouchon (2000, 2600) selon la revendication 4, dans lequel ledit bouchon (2000, 2600) peut être détecté en utilisant la fluoroscopie.

6. Le bouchon (2000, 2600) selon la revendication 4, dans lequel ladite partie tubulaire (2050) perforée et lesdites première et deuxième pluralités de doigts (2008, 2010 ; 2608, 2610) sont formées à partir d'un corps unitaire.

7. Le bouchon (2000, 2600) selon la revendication 4, dans lequel la structure tubulaire (2050) perforée et lesdits doigts sont formés d'un matériau élastique.

8. Le bouchon (2000, 2600) selon la revendication 7, dans lequel ladite structure tubulaire (2050) perforée et lesdits doigts sont formés de nitinol.

9. Le bouchon (2000, 2600) selon la revendication 4, dans lequel au moins un doigt desdites pluralités de doigts (2008, 2010 ; 2608, 2610) peut être positionné pour s'étendre sensiblement radialement à l'écart dudit passage longitudinal.

10. Le bouchon (2000, 2600) selon la revendication 4, dans lequel au moins un doigt de ladite pluralité de doigts (2608, 2610) présente une structure de marqueur.

11. Le bouchon (2000, 2600) selon la revendication 10, dans lequel ladite structure de marqueur est radio-opaque.

12. Le bouchon (2000, 2600) selon la revendication 10, dans lequel ladite structure de marqueur est formée de bandes de marqueur.

13. Le bouchon (2000, 2600) selon la revendication 15, dans lequel ledit doigt comprend une partie d'extrémité et ladite bande de marqueur est sertie sur ladite partie d'extrémité.

14. Le bouchon (2000, 2600) selon la revendication 10, dans lequel ladite structure de marqueur est formée d'un rivet.

15. Le bouchon (2000, 2600) selon la revendication 4, dans lequel au moins un doigt d'au moins l'une desdites pluralités de doigts présente un réceptacle pour dispositif de rétention.

16. Le bouchon (2000, 2600) selon la revendication 15, dans lequel ledit réceptacle pour dispositif de rétention est une ouverture pour goupille de verrouillage.

17. Le bouchon (2000, 2600) selon la revendication 16, dans lequel ledit réceptacle pour dispositif de rétention est un couvercle de cône de nez.

18. Le bouchon (2000, 2600) selon la revendication 4, dans lequel au moins un doigt (2008, 2010) d'au moins l'une desdites pluralités de doigts présente une partie d'extrémité pointue, localement distante d'un axe central (2004).

19. Le bouchon (2000, 2600) selon la revendication 4, dans lequel au moins un doigt d'au moins l'une desdites pluralités de doigts présente une partie d'extrémité à barbes, localement distante d'un axe central.

20. Le bouchon (2000, 2600) selon la revendication 4, dans lequel au moins un doigt (2008, 2010), d'au moins l'une desdites pluralités de doigts, est incurvé, ladite courbe étant concave, orientée vers un plan perpendiculaire à un axe central (2004) et passant sensiblement entre lesdites premières et deuxième pluralités de doigts.

21. Le bouchon (2000, 2600) selon la revendication 4, dans lequel ladite partie tubulaire (2050) perforée définit une section transversale, sensiblement ronde lorsqu'on l'observe dans une direction dudit axe longitudinal.

22. Le bouchon (2000, 2600) selon la revendication 4, dans lequel ladite partie tubulaire (2050) perforée définit une section transversale, sensiblement elliptique lorsqu'on l'observe en une direction dudit passage longitudinal.

23. Le bouchon (2000, 2600) selon la revendication 4, dans lequel pratiquement la totalité desdits doigts (2008, 2010 ; 2608, 2610) d'au moins l'une desdites pluralités sont d'une longueur sensiblement similaire.

24. Le bouchon (2000, 2600) selon la revendication 4, dans lequel différents doigts, parmi les doigts (2008, 2010 ; 2608, 2610) d'au moins l'une desdites pluralités, sont de longueurs différentes.

25. Le bouchon (2000, 2600) selon la revendication 4, dans lequel au moins un doigt (2008, 2010 ; 2608, 2610) d'au moins l'une desdites pluralités de doigts présente une rigidité en flexion différente sur sa longueur.

26. Le bouchon (2000, 2600) selon la revendication 4, dans lequel au moins un doigt (2008, 2010 ; 2608, 2610) d'au moins l'une desdites pluralités de doigts présente une épaisseur différente sur sa longueur.

27. Le bouchon (2000, 2600) selon la revendication 4, dans lequel au moins un doigt (2008, 2010 ; 2608, 2610) d'au moins l'une desdites pluralités de doigts présente une largeur différente sur sa longueur.

28. Le bouchon (2000, 2600) selon la revendication 4, dans lequel au moins un doigt (2008, 2010 ; 2608, 2610) d'au moins l'une desdites pluralités de doigts présente une partie d'extrémité libre, comprenant une structure d'extrémité configurée pour faciliter une rétention désolidarisable dudit doigt, par un dispositif de fourniture de bouchon.

29. Le bouchon (2000, 2600) selon la revendication 4, comprenant en outre une âme élastique entre des doigts adjacents parmi lesdits doigts (2008, 2010 ; 2608, 2610).

30. Le bouchon (2000, 2600) selon la revendication 29, dans lequel âme est formée de silicone.

31. Le bouchon (2000, 2600) selon la revendication 4, dans lequel au moins un doigt (2008, 2010 ; 2608, 2610) d'au moins l'une desdites pluralités de doigts présente une partie d'extrémité proche d'un axe central, afin de supporter ladite structure de bouchage.

32. Le bouchon (2000, 2600) selon la revendication 4, dans lequel au moins un doigt (2008, 2010 ; 2608, 2610) d'au moins l'une desdites pluralités de doigts présente une partie d'extrémité distante d'un axe central, pour supporter ladite structure de bouchage.

33. Le bouchon (2000, 2600) selon la revendication 32, dans lequel ladite partie d'extrémité présente une structure support (2613) avec laquelle est effectuée la fixation de ladite structure de bouchage (2607).

34. Le bouchon (2000, 2600) selon la revendication 4, dans lequel ladite partie tubulaire (2050) perforée présente au moins une structure support avec laquelle est effectuée la fixation de ladite structure de bouchage.

35. Le bouchon (2000, 2600) selon la revendication 4, dans lequel ladite structure de bouchage (2607, 2606) est élastique.

36. Le bouchon (2000, 2600) selon la revendication 4, dans lequel ladite structure de bouchage (2607, 2606) peut être dépliée.

37. Le bouchon (2000, 2600) selon la revendication 4, dans lequel ladite structure de bouchage (2607, 2606) comprend du matériau polymère.

38. Le bouchon (2000, 2600) selon la revendication 4, dans lequel ladite structure de bouchage comprend un tissu.

39. Le bouchon (2000, 2600) selon la revendication 4, dans lequel ladite structure de bouchage (2606) est cousue sur un cadre (2002).

40. Le bouchon (2000, 2600) selon la revendication 4, dans lequel ladite structure de bouchage (2606) présente une ouverture pour fil de guidage, à travers laquelle peut passer un fil de guidage.

41. Le bouchon (2000, 2600) selon la revendication 40, dans lequel ladite ouverture pour fil de guidage peut se fermer pratiquement automatiquement, après avoir enlevé ledit fil de guidage.

42. Le bouchon (2000, 2600) selon la revendication 39, dans lequel ledit cadre (2002) peut être inséré dans un tube de fourniture en étendant lesdits doigts en une direction sensiblement parallèle audit passage longitudinal.

43. Le bouchon (2000, 2600) selon la revendication 4, dans lequel ladite partie tubulaire (2050) perforée peut se conformer à un périmètre d'un trou dans une paroi d'une cavité corporelle d'un patient.

44. Le bouchon (2000, 2600) selon la revendication 4, dans lequel ladite partie tubulaire (2050) perforée peut se contracter longitudinalement lorsqu'elle s'expanse radialement.

45. Le bouchon (2000, 2600) selon la revendication 4, dans lequel ladite partie tubulaire (2050) perforée est déformable plastiquement.

46. Le bouchon (2000, 2600) selon la revendication 45, dans lequel ladite partie tubulaire (2050) perforée est formée d'acier inoxydable.

47. Le bouchon (2000, 2600) selon la revendication 46, dans lequel ladite partie tubulaire (2050) est formée de tantale.

48. Le bouchon (2000, 2600) selon la revendication 45, dans lequel ladite partie tubulaire (2050) perforée peut être conformée à un périmètre d'un trou dans une paroi d'une cavité corporelle d'un patient, en utilisant un ballon pour obtenir l'expansion de ladite partie tubulaire perforée.

49. Le bouchon (2000, 2600) selon la revendication 45, dans lequel ladite partie tubulaire perforée peut se contracter longitudinalement lorsqu'elle s'expanse radialement.

50. Le bouchon (2000, 2600) selon la revendication 1, dans lequel ladite partie tubulaire (2050) perforée et lesdits doigts sont formés d'un matériau élastique.

51. Le bouchon (2000, 2600) selon la revendication 50, dans lequel ladite partie tubulaire perforée et lesdits doigts sont formés de nitinol.

52. Le bouchon (2000, 2600) selon la revendication 1, dans lequel au moins l'un desdits doigts (2008, 2010) s'étend sensiblement radialement à l'écart d'un axe central.

53. Le bouchon (2000, 2600) selon la revendication 1, dans lequel au moins l'un desdits doigts (2008, 2010; 2608, 2610) présente une structure de marqueur.

54. Le bouchon (2000, 2600) selon la revendication 53, dans lequel ladite structure de marqueur est radio-opaque.

55. Le bouchon (2000, 2600) selon la revendication 53, dans lequel ladite structure de marqueur est une bande de marqueur.

56. Le bouchon (2000, 2600) selon la revendication 55, dans lequel ledit doigt présente une partie d'extrémité et ladite bande de marqueur est sertie sur ladite partie d'extrémité.

57. Le bouchon (2000, 2600) selon la revendication 53, dans lequel ladite structure de marqueur est un rivet.

58. Le bouchon (2000, 2600) selon la revendication 1, dans lequel au moins l'un desdits doigts (2008, 2010; 2608, 2610) présente une partie d'extrémité pointue, située à distance d'un axe central.

59. Le bouchon (2000, 2600) selon la revendication 1, dans lequel au moins l'un desdits doigts présente une partie d'extrémité à barbes, placée à distance d'un axe central.

60. Le bouchon (2000, 2600) selon la revendication 1, dans lequel au moins l'un desdits doigts (2008, 2010; 2608, 2610) est incurvé, ladite courbe étant concave, vers un plan perpendiculaire à un axe central et passant par ladite partie tubulaire (2050) perforée.

61. Le bouchon (2000, 2600) selon la revendication 1, dans lequel ladite partie tubulaire (2050) perforée définit une section transversale, sensiblement ronde lorsqu'on l'observe dans une direction dudit passage longitudinal.

62. Le bouchon (2000, 2600) selon la revendication 1, dans lequel ladite partie tubulaire (2050) perforée définit une section transversale, sensiblement elliptique lorsqu'on l'observe dans une direction dudit passage longitudinal.

63. Le bouchon (2000, 2600) selon la revendication 1, dans lequel pratiquement la totalité desdits doigts (2008, 2010; 2608, 2610) sont d'une longueur sensiblement similaires.

64. Le bouchon (2000, 2600) selon la revendication 1, dans lequel des doigts différents, parmi les doigts (2008, 2010; 2608, 2610), sont de longueurs différentes.

65. Le bouchon (2000, 2600) selon la revendication 1, dans lequel au moins l'un desdits doigts (2008, 2010; 2608, 2610) présente une rigidité en flexion différente sur sa longueur.

66. Le bouchon (2000, 2600) selon la revendication 1, dans lequel au moins l'un desdits doigts (2008, 2010; 2608, 2610) présente une épaisseur différente sur sa longueur.

67. Le bouchon (2000, 2600) selon la revendication 1, dans lequel au moins l'un desdits doigts (2008, 2010; 2608, 2610) présente une largeur différente sur sa longueur.

68. Le bouchon (2000, 2600) selon la revendication 1, dans lequel au moins l'un desdits doigts (2008, 2010; 2608, 2610) présente une partie d'extrémité libre, comprenant une structure d'extrémité, configurée pour faciliter une rétention désolidarisable dudit doigt par un dispositif de fourniture de bouchon.

69. Le bouchon (2000, 2600) selon la revendication 1 comprenant une âme élastique entre des doigts adjacents parmi lesdits doigts.

70. Le bouchon (2000, 2600) selon la revendication 69, dans lequel l'âme est formée de silicone.

71. Le bouchon (2000, 2600) selon la revendication 1, dans lequel au moins l'un desdits doigts présente une partie d'extrémité proche d'un axe central, afin de supporter ladite structure de bouchage.

72. Le bouchon (2000, 2600) selon la revendication 71, dans lequel ladite partie d'extrémité présente une structure support, servant à la fixation de ladite structure de bouchage.

73. Le bouchon (2000, 2600) selon la revendication 1, dans lequel ladite partie tubulaire (2050) perforée présente au moins une structure support, pour supporter ladite structure de bouchage.

74. Le bouchon (2000, 2600) selon la revendication 1, dans lequel ladite structure de bouchage est élastique.

75. Le bouchon (2000, 2600) selon la revendication 1, dans lequel ladite structure de bouchage peut être déployée.

76. Le bouchon (2000, 2600) selon la revendication 1, dans lequel ladite structure de bouchage est formée d'un matériau polymère.

77. Le bouchon (2000, 2600) selon la revendication 1, dans lequel ladite structure de bouchage comprend un tissu.

78. Le bouchon (2000, 2600) selon la revendication 1, dans lequel ladite structure de bouchage (2606) est cousue sur ledit cadre (2002).

79. Le bouchon (2000, 2600) selon la revendication 1, dans lequel ladite structure de bouchage (2606, 2607) présente une ouverture pour fil de guidage, à travers laquelle peut passer un fil de guidage.

80. Le bouchon (2000, 2600) selon la revendication 79, dans lequel ladite ouverture pour fil de guidage peut se refermer pratiquement automatiquement, après avoir enlevé ledit fil de guidage.

81. Le bouchon (2000, 2600) selon la revendication 1, dans lequel un cadre (2002) peut être inséré dans un tube de fourniture, en étendant lesdits doigts dans une direction sensiblement parallèle audit passage longitudinal.

82. Le bouchon (2000, 2600) selon la revendication 1, dans lequel ladite partie tubulaire (2050) perforée peut se conformer à un périmètre, d'un défaut dans une paroi d'une cavité corporelle d'un patient.

83. Le bouchon (2000, 2600) selon la revendication 1, dans lequel ladite partie tubulaire (2050) perforée peut se contracter longitudinalement lorsqu'elle s'expanse radialement.

84. Le bouchon (2000, 2600) selon la revendication 1, dans lequel ladite partie tubulaire (2050) perforée est déformable plastiquement.

85. Le bouchon (2000, 2600) selon la revendication 84, dans lequel ladite partie tubulaire (2050) perforée comprend de l'acier inoxydable.

86. Le bouchon (2000, 2600) selon la revendication 84, dans lequel ladite partie tubulaire (2050) perforée comprend du tantale.

87. Le bouchon (2000, 2600) selon la revendication 84, dans lequel ladite partie tubulaire (2050) perforée peut être conformée à un périmètre d'un trou dans une paroi d'une cavité corporelle d'un patient, en utilisant un ballon pour expanser ladite partie tubulaire perforée.

88. Le bouchon (2000, 2600) selon la revendication 84, dans lequel ladite partie tubulaire (2050) perforée peut se contracter longitudinalement lorsqu'elle s'expanse radialement.
